(19) Europäisches Patentamt
European Patent Office
Office européen des brevets

(11) Publication number: 0 129 407
A2

# (12) EUROPEAN PATENT APPLICATION

(21) Application number: 84304005.6

(22) Date of filing: 14.06.84

(51) Int. Cl.³: C 07 D 275/02
A 01 N 47/36

(30) Priority: 20.06.83 US 506176

(43) Date of publication of application:
27.12.84 Bulletin 84/52

(84) Designated Contracting States:
BE CH DE FR GB IT LI LU NL SE

(71) Applicant: ELI LILLY AND COMPANY
307, East McCarty Street
Indianapolis Indiana 46285(US)

(72) Inventor: Hackler, Ronald Ervin
419, Woodland West Drive, Rt. 7
Greenfield Indiana 46140(US)

(74) Representative: Tapping, Kenneth George et al,
Lilly Industries Limited Patent Department Erl Wood Manor
Windlesham Surrey, GU20 6PH(GB)

(54) **Improvements in and relating to (3-aryl-5-isothiazolyl)ureas.**

(57) Novel (3-aryl-5-isothiazolyl)-ureas, which are useful as aquatic algicides, terrestrial herbicides, and aquatic herbicides, are described herein. These ureas are prepared from the corresponding 5-amino-3-arylisothiazoles with a ureido forming reagent. Further provided are methods of use and formulations comprising these urea products.

EP 0 129 407 A2

0129409
EPO-DG 1
Rec.
20 JUN 1984



# IMPROVEMENTS IN AND RELATING TO (3-ARYL-5-ISOTHIAZOLYL)UREAS

This invention concerns a class of novel N'-(3-aryl-5-isothiazolyl)-N-substituted-ureas, which are useful as aquatic algicides, terrestrial herbicides, and aquatic herbicides.

The use of herbicides, both terrestrial and aquatic, and aquatic algicides to control selectively the growth of unwanted vegetation and algae is well established. However, a number of problems are associated with the continued use of chemical herbicides and algicides, including environmental pollution, crop tolerance, weed resistance, as well as economic considerations. Accordingly, the search continues for new herbicides and algicides which are economical and which are selectively toxic to unwanted vegetation.

A variety of isothiazolylureas are known in the art. For example, U.S. Patent 3,622,593 teaches an unsubstituted isothiazole ring attached to a urea, wherein the urea is also substituted with methyl groups. British Patent 1,153,186 describes the preparation of 6-[5'-(lower)alkyl-3'-arylisothiazole-4'-carboxamido]-penicillanic acids, including intermediate 3-aryl-4-substituted-5-(lower)alkylisothiazoles.

This invention concerns isothiazolylureas of the formula

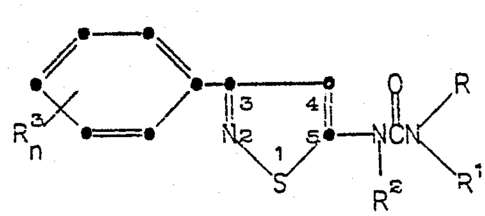

IX

wherein

R and $R^1$ are $C_1$-$C_6$ alkyl, $C_3$-$C_6$ alkenyl, or $C_1$-$C_2$ alkoxy, provided that one of R or $R^1$ is methyl;

$R^2$ is hydrogen or $C_1$-$C_3$ alkyl;

$R^3$ is hydrogen, halo, $C_1$-$C_2$ alkoxy, or $C_1$-$C_6$ alkyl; and

n is 1 or 2;

or an agriculturally- or aquatically-acceptable acid-addition salt thereof.

The compounds of formula IX are prepared by reacting a 5-aminoisothiazole of the formula

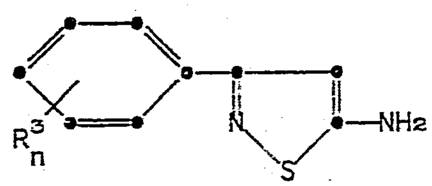

V

wherein $R^3$ and n are defined as before, with a ureido forming reagent, to provide the compounds of formula IX wherein $R^2$ is hydrogen;

followed by alkylation with

$$R^{2'}Y$$

wherein $R^{2'}$ is $C_1$-$C_3$ alkyl and Y is bromo, chloro, fluoro or iodo, to provide the compounds of formula IX wherein $R^2$ is $C_1$-$C_3$ alkyl; and

optionally followed by salification to form the corresponding acid-addition salt.

Examples of suitable ureido forming reagents are

a) RNCO,

b) $RR^1N\underset{\underset{O}{\|}}{C}V$,

c) $COV_2$ and $HNRR^1$,

d) V-phenylformate and $HNRR^1$, and

e) V-phenylformate and hydroxylamine, followed by alkylating with dimethyl sulfate,

wherein R and $R^1$ are defined as before, and V is chloro or bromo.

The (3-aryl-5-isothiazolyl)ureas of formula IXA, X or XI (defined below), or an agriculturally- or aquatically-acceptable acid addition salt thereof, will normally be sold as a herbicidal or algicidal formulation together with one or more agriculturally- or aquatically-acceptable carriers or diluent therefor. Formulations comprising one or more other compatible herbicides or algicides are also contemplated.

Additionally provided by the invention is a method for controlling the growth of unwanted terrestrial vegetation which comprises contacting the vegetation to be controlled or the soil in which the vegetation is growing with a herbicidally-effective amount of a (3-aryl-5-isothiazolyl)urea of formula X, defined hereinafter.

Also provided by the invention is a method of controlling the growth of unwanted aquatic vegetation which comprises contacting the vegetation to be controlled or the water in which the vegetation is growing with an aquatically herbicidally-effective amount of a compound of formula XI, defined hereinafter.

The invention also provides a method of controlling the growth of aquatic algae which comprises contacting the algae to be controlled or the water in which the algae is growing with an algicidally-effective amount of a compound of formula IXA, defined below.

In addition to the isothiazolylurea compounds of formula IX, there is also provided a preferred group of compounds of the formula IX wherein:

R and $R^1$ are $C_1$-$C_4$ alkyl or $C_1$-$C_2$ alkoxy, provided that one of R or $R^1$ is methyl;

$R^2$ is hydrogen;

$R^3$ is hydrogen, halo or methyl; and

n is 1.

Even more preferred compounds of formula IX are those wherein:

R is methyl; $R^1$ is methoxy or methyl;

$R^2$ is hydrogen; and

$R^3$ is 4-fluoro.

Most preferred compounds of formula IX include:

N'-[3-(4-fluorophenyl)-5-isothiazolyl]-N-methoxy-N-methylurea;

N'-[3-(4-fluorophenyl)-5-isothiazolyl]-N,N-dimethylurea; and

N'-[3-(4-fluorophenyl)-5-isothiazolyl]-N-methoxy-N-methylurea monohydrochloride.

Other compounds, which are preferred for use in the various methods, include:

N'-[3-(4-methylphenyl)-5-isothiazolyl]-N,N-dimethylurea;

N'-(3-phenyl-5-isothiazolyl)-N-methoxy-N-methylurea;

N'-[3-(4-chlorophenyl)-5-isothiazolyl]-N-methyl-N-(1-methylethyl)urea;

N'-[3-(4-bromophenyl)-5-isothiazolyl]-N,N-dimethylurea;

N'-[3-(4-chlorophenyl)-5-isothiazolyl]-N,N-dimethylurea;

N'-(3-phenyl-5-isothiazolyl)-N,N-dimethylurea;

N'-[3-(4-fluorophenyl)-5-isothiazolyl]-N-ethyl-N-methylurea;

N'-[3-(4-fluorophenyl)-5-isothiazolyl]-N'-ethyl-N-methyl-N-(1-methylethyl)urea;

N'-[3-(4-fluorophenyl)-5-isothiazolyl]-N',N,N-trimethylurea;

N'-[3-(4-methylphenyl)-5-isothiazolyl]-N-methoxy-N-methylurea;

N'-[3-(4-chlorophenyl)-5-isothiazolyl]-N-methyl-N-(1-methylethyl)urea;

N'-[3-(4-chlorophenyl)-5-isothiazolyl]-N-methoxy-N-methylurea;

N-[3-(4-methylphenyl)-5-isothiazolyl]-N-ethyl-N-methylurea;

N'-[3-(4-chlorophenyl)-5-isothiazolyl]-N-methyl-N-(1-methylpropyl)urea;

N'-(3-phenyl-5-isothiazolyl)-N-methyl-N-(1-methylethyl)urea; and

N'-[3-(4-chlorophenyl)-5-isothiazolyl]-N-methyl-N-(1-methylpropyl)urea monohydrochloride.

The following defines the various terms used in this application.

The term "$C_1$-$C_6$ alkyl" refers to the straight and branched aliphatic radicals of one to six carbon atoms including ethyl, propyl, isopropyl (1-methyl ethyl), butyl, methyl, isobutyl (2-methylpropyl), sec-butyl (1-methylpropyl), tert-butyl (1,1-dimethylethyl), pentyl, isopentyl (3-methylbutyl), sec-pentyl (1-methylbutyl), 1,1-dimethylpropyl, 1,2-dimethylpropyl, neopentyl (2,2-dimethylpropyl), hexyl, isohexyl (4-methylpentyl), sec-hexyl (1-methylpentyl), 2-methylpentyl, 3-methylpentyl, 1,1-dimethylbutyl, 2,2-dimethylbutyl, 3,3-dimethylbutyl, 1,2-dimethylbutyl, 1,3-dimethylbutyl, 1,2,2-trimethylpropyl, 1,1,2-trimethylpropyl, and the like. The terms "$C_1$-$C_4$ alkyl", and "$C_1$-$C_3$ alkyl" are also included in this definition.

The term "$C_3$-$C_6$ alkenyl" refers to unsaturated aliphatic radicals of three to six carbon atoms, wherein one carbon to carbon double bond exists in the radical, and includes propenyl, 1-butenyl, 2-butenyl, 2-methylpropenyl, 1-pentenyl, 2-pentenyl, 3-pentenyl, 3-methyl-1-butenyl, 2-methyl-2-butenyl, 1-hexenyl, 2-hexenyl, 3-hexenyl, 2,3-dimethyl-2-butenyl, and the like.

The term "$C_1$-$C_2$ alkoxy" refers to the aliphatic radicals of one to two carbon atoms attached to the remainder of the molecule by an oxygen atom, such as methoxy, and ethoxy.

The term "halo" refers to bromo, chloro, fluoro, and iodo.

## Preparation of Ureas of Formula IX

The process for making the (3-aryl-5-isothiazolyl)ureas of formula IX is outlined in the following schematic:

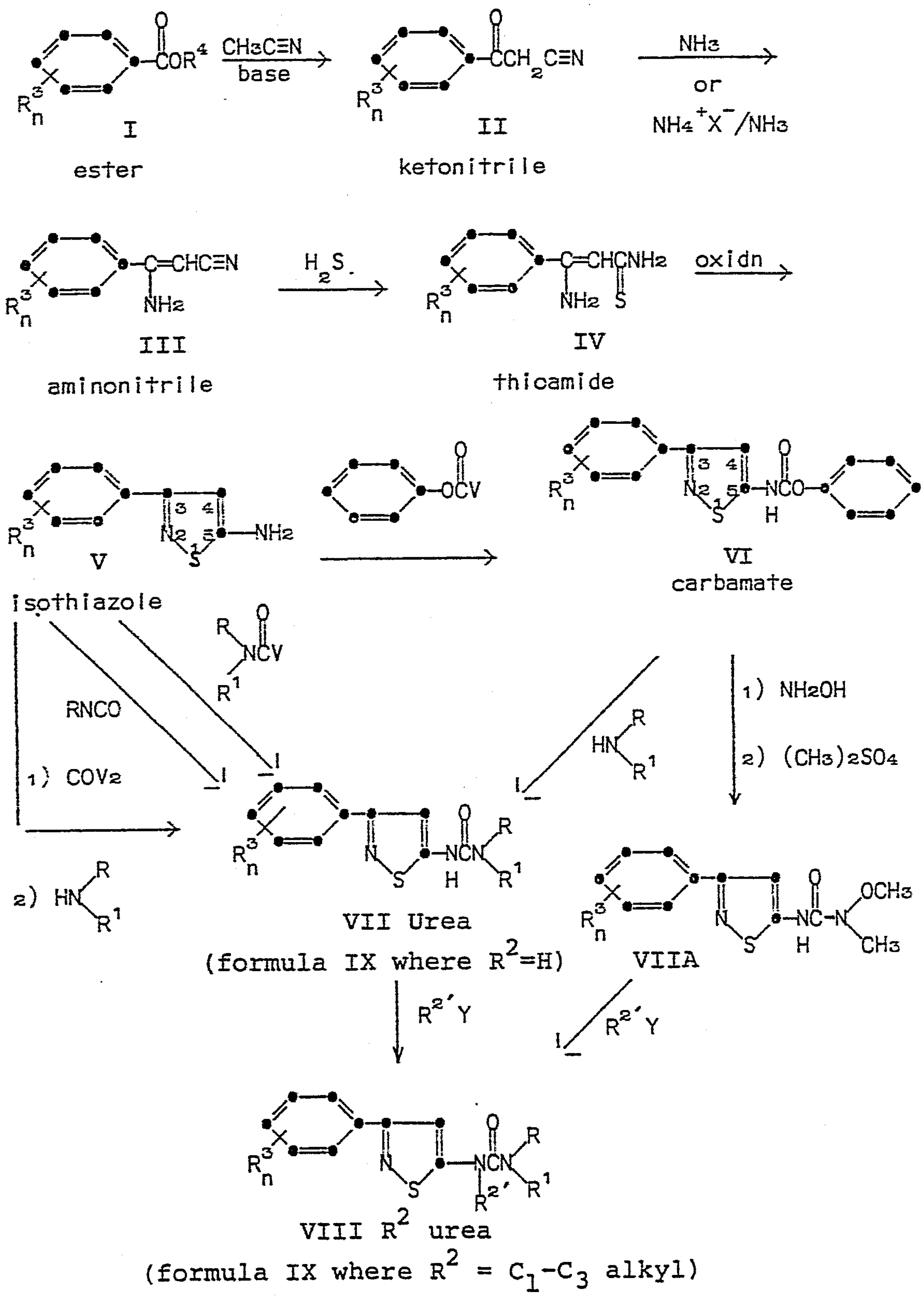


I ester

II ketonitrile

III aminonitrile

IV thioamide

V isothiazole

VI carbamate

VII Urea

(formula IX where $R^2$=H)

VIIA

VIII $R^2$ urea

(formula IX where $R^2$ = $C_1$-$C_3$ alkyl)

wherein R, $R^1$, and $R^3$ are defined as above, and $R^{2'}$ is $C_1$-$C_3$ alkyl;

$R^4$ is $C_1$-$C_6$ alkyl or phenyl;

V is chloro or bromo;

X is nitrate, acetate, formate, bromide, chloride, or fluoride; and

Y is bromo, chloro, fluoro, or iodo.

A substituted ester of formula I is converted to the ketonitrile of formula II using acetonitrile and a base. The ketonitrile of formula II is then treated with ammonia or an ammonium salt-ammonia mixture to form the aminonitrile of formula III. Subsequently, thiation of this aminonitrile of formula III is completed using hydrogen sulfide. Upon thiation a thioamide of formula IV is formed, which is then oxidized to facilitate ring closure, resulting in the 3-substituted-5-aminoisothiazole of formula V.

Once the isothiazole of formula V is formed, it is reacted with a halogenated phenylformate, usually chloro, to generate a carbamate intermediate of formula VI. This intermediate of formula VI is subsequently aminated to produce the desired urea compound of formula VII.

Alternatively, the urea compound of formula VII can be made from (a) the reaction of a 5-aminoisothiazole of formula V and the appropriate alkylisocyanate in an inert organic solvent or from (b) the reaction of the isothiazole of formula V with phosgene to form an isocyanate and then reacting the isocyanate with the appropriate amine or from (c) the reaction of a 5-aminoisothiazole of formula V with a carbamoyl halide.

A certain group of urea compounds of formula VII, such as the N-methyl-N-methoxy derivatives of formula VIIA, can be prepared by the alkylation of the corresponding hydroxyl urea.

Other, more-substituted ureas of formula VIII can also be prepared from already-made ureas of formulae VII and VIIA by alkylation.

## Compound I - Ester

Compound I, the substituted ester, can be obtained by methods well-known in the art. These include the preparation of the ester from the corresponding phenyl carboxylic acid, 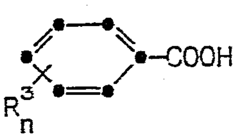 , wherein $R^3$ and n are defined above, and the appropriate alcohol, $R^4OH$, wherein $R^4$ is defined above, under acidic conditions. Typical acids used include concentrated sulfuric acid, dry hydrogen chloride, and boron trifluoride etherate, with boron trifluoride etherate preferred. Preferred alcohols are methanol and ethanol, wherein $R^4$ is methyl or ethyl, with the most preferred being methanol. The resulting preferred ester will be the methyl carboxylate. Preferred carboxylic acid groups, $R^3$, are listed above. Usually, to shift the equilibrium toward the ester, a large excess of alcohol is used with elevated temperatures, but the temperature can be from about ambient temperature to about the reflux temperature of the alcohol. Alternatively, the ester can be removed upon formation.

A more preferred preparation of the ester involves using the corresponding acid chloride,

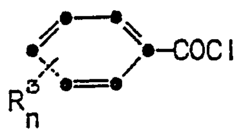, and the appropriate alcohol, $R^4OH$.

Esterification of the acid chloride is often carried out in the presence of a base, such as pyridine, aqueous sodium hydroxide, or others, under conditions described as the Schotten-Baumann technique. However, sometimes the use of the above-listed bases is unnecessary. (Typically, the acid chloride is prepared from its carboxylic acid using thionyl chloride, phosphorus oxychloride, oxalyl chloride, or phosphorus pentachloride.) Acid anhydrides can also undergo alcoholysis to form an ester, as do the acid chlorides.

The process called transesterification allows conversion of one ester to another, catalyzed by acidic or basic conditions. The acid can be concentrated sulfuric acid, dry hydrogen chloride, and the like, while the base can be an alkoxide ion, and the like. To shift the equilibrium to form the new ester, a large excess of the appropriate alcohol is used or the desired ester is removed upon formation.

The intermediates of formula I can be isolated, if desired, by typical techniques, such as extraction or recrystallization. However, isolation is not necessary when an intermediate is going to be used in a following reaction. Compound I is typically isolated by being washed with water and then extracted with ether and dried over magnesium sulfate.

Compound II - Ketonitrile

The ketonitrile, compound II, is formed from the substituted ester of formula I using a base condensation reaction with acetonitrile. The base present can be sodium metal, sodium ethoxide, sodium methoxide, sodium hydride, and the like. Sodium hydride is the base of choice and is usually dispersed in mineral oil. The substituted ester of formula I is allowed to react with from about one to about two equivalents of acetonitrile in the presence of the base and a solvent at the reflux temperature of the reaction mixture. The reaction continues for a time sufficient to bring about substantial completion, usually from about 4 hours to about 24 hours. Suitable solvents include tetrahydrofuran (THF), ether, toluene, benzene, ethanol, methanol, and the like, with THF the preferred solvent.

A preferred method for preparing Compound II is slurrying sodium hydride in THF and then adding the appropriate ester and acetonitrile, while the slurry is maintained at a temperature from about 60 to about 65°C. After the addition is complete, the mixture is heated to reflux for about 16 to about 24 hours.

The intermediates of formula II can be isolated, if desired, by typical techniques. The ketonitrile, Compound II, is formed in the reaction mixture as the sodium salt. Then the solvent is removed *in vacuo* before the salt is dissolved in water. Any residual mineral oil is washed out with hexane, and the free ketonitrile is obtained by acidifying with a mineral acid, such as hydrochloric acid and the like,

and extracting into ether. Any necessary crystallization can be done with ethanol, methylene chloride, cyclohexane, hexane, toluene, or appropriate mixtures thereof.

Compound III - Aminonitrile

Once the ketonitrile of formula II is formed, it can be converted to the aminonitrile, Compound III, by using a source of ammonia. Using ammonia under pressure provides a source of ammonia, with the pressure being generated by the reaction itself run in a sealed vessel. The reaction is heated at temperatures from about 100 to about 150°C for anywhere from about 4 to about 24 hours. Preferably, the reaction is conducted at about 150°C for about 16 hours. Although an organic solvent can be used in the reaction, such as ethanol, benzene, toluene, methanol, THF, and the like, with ethanol being preferred, such a solvent is not essential to the reaction. It is, however, necessary to use at least one equivalent of ammonia, but an excess of ammonia can be used if desired.

The preferred source of ammonia utilizes an ammonium salt and ammonia under atmospheric pressure at temperatures from about ambient temperature to about the reflux temperature of the solvent used. The ketonitrile, Compound II, is mixed in an inert organic solvent that already contains the ammonium salt. Typical solvents include ethanol, methanol, THF, and the like, with ethanol being preferred. Typical ammonium salts include ammonium chloride, ammonium bromide, ammonium

acetate, ammonium formate, ammonium nitrate, and the like, with ammonium nitrate being preferred, but, ammonium sulfate cannot be used. The amount of ammonium salt can be varied and an excess can be used; however, it is preferable to use less than or equal to one equivalent of the salt in the reaction. The resulting mixture of solvent and ammonium salt is then warmed to the desired temperature, which is usually the reflux temperature of the solvent, and ammonia is bubbled in. The reaction runs from about 4 to about 24 hours, with the preferred time being from about 16 to 24 hours.

Alternatively, Compound II may be reacted with one to two equivalents of ammonia with an ammonium salt added, all under pressure, at a temperature of about 25 to about 80°C for about 4 to about 24 hours.

A more preferred method of preparing Compound III uses ammonium nitrate as the desired ammonium salt, uses ethanol as the solvent, and is run for about 16 hours at the reflux temperature of ethanol, which is about 80°C.

Another route to Compound III is described in the literature; see Netherlands Patent 6,608,094, J. Prakt. Chem., 52, 110 (1895), and J. Med. Chem., 18, 441 (1975). This route involves reacting acetonitrile with an appropriate base, such as sodium metal, sodium hydride, or sodium amide in the presence of an aromatic nitrile. After condensation occurs and the reaction is treated with water, Compound III is isolated upon work-up.

Compound III, the aminonitrile, can be isolated, if desired, by removing the excess ammonia and

solvent *in vacuo*, and then extracting with hexane, methylene chloride, ethyl acetate, ether, and the like. The mixture containing the product can also be dried over magnesium sulfate, and then recrystallized. Typical solvents used for crystallization are toluene, hexane, cyclohexane, and the like.

### Compound IV - Thioamide

Compound IV, a thioamide, can be made under pressure by following a procedure similar to that outlined in J. Goerdeler and H.W. Pohland, *Chem. Ber.*, *94*, 2950 (1961) and British Patent 1,153,186 (Netherlands Patent 6,608,094).

The aminonitrile, Compound III, is reacted with hydrogen sulfide under pressure; the pressure being generated by the reaction itself. This reaction is run in an organic solvent, such as methylene chloride, chloroform, and the like, with methylene chloride being preferred. A small amount of a base-catalyst (0.5-2%) is used to facilitate reaction and includes such bases as: triethylamine and potassium hydroxide, with the latter preferred. The temperature of the reaction can be from about 25 to about 100°C, with about 60°C being preferred, while the reaction can be allowed to continue from about 16 hours to about 72 hours, with about 48 hours being preferred.

A more preferred method of making the thioamide of formula IV with hydrogen sulfide utilizes a temperature at about 60°C for about 48 hours, with methylene chloride as the solvent, and potassium hydroxide as the catalyst.

Alternatively, the thioamide of formula IV can be prepared at atmospheric pressure by reacting the aminonitrile of formula III in an inert organic solvent such as pyridine, ethanol, toluene, benzene, and the like, pyridine being preferred, with hydrogen sulfide in the presence of a base-catalyst, such as triethylamine. Typically, this type of reaction is run at a temperature from about 25 to about 100°C.

Another route, even more preferred, involves using a phase-transfer catalyst (PTC) at about one to two atmospheres of pressure (atm), following a procedure similar to that outlined in L. Cassar, S. Panossian, and C. Giordano, Synthesis, 917 (1978). The aminonitrile of formula III is dissolved in an inert organic solvent, such as benzene, toluene, 1,2-dichlorobenzene, diphenyl ether, and the like. (The use of a solvent can be avoided if a liquid aminonitrile is utilized.) Then an aqueous solution of sulfide ion (usually sodium sulfide nonahydrate is used to supply the sulfide ion) and the PTC are added. The use of dilute aqueous sulfide solutions are preferred, allowing the reaction to proceed more rapidly. The mixture is then heated to about 35-80°, preferably 70°C, under an atmosphere of hydrogen sulfide at a pressure of about 1-2 atm for about 4 to about 48 hours. Typical PTCs include tetrabutylammonium chloride, tetrabutylammonium bromide, tricaprylmethylammonium chloride, dibenzo-18-crown-6, and the like. The preferred PTC is tetrabutylammonium chloride (TBAC).

The thioamide of formula IV, prepared by the first route, can be isolated, if desired, by removing the excess hydrogen sulfide and solvent and then crystallizing it from other solvents, such as ethanol, toluene, benzene, ethyl acetate, chloroform, hexane, and the like. Once the preparation of the thioamide is completed using the PTC or second route, the water layer is removed, and the organic layer is isolated. The product contained in the organic layer is precipitated, and can be recrystallized from solvents, such as methylene chloride, ether, benzene, hexane, ethyl acetate, and the like. Any excess solvent can be removed and/or dried before the desired product is obtained.

## Compound V - Isothiazole

Ring closure of the thioamide of formula IV to form the isothiazole of formula V is facilitated by using an oxidizing agent, such as iodine, bromine, hydrogen peroxide, sodium hypochlorite, chloramine, chloramine T, chlorine, persalts such as potassium or ammonium persulfate, and the like. Preferred oxidizing agents are iodine and hydrogen peroxide. First, the thioamide is dissolved in an inert organic solvent, such as ether, benzene, ethanol water, and the like, and then the oxidizing agent is added.

After cyclization of the thioamide of formula IV, the isothiazole of formula V can be isolated, if desired, as follows. The organic layer containing the product can be dried and then the solvent removed.

Crystallization can be done using toluene, ethanol, cyclohexane, acetone, hexane, methylene chloride, chloroform, ether, and mixtures thereof, and the like.

When impure thioamide of formula IV is used in the cyclization step, the isothiazole product of formula V is best purified by extracting it from the organic solvent with a dilute mineral acid, washing the aqueous solution with an organic solvent, such as methylene chloride, and the like. The isothiazole of formula V is then freed with sodium or potassium hydroxide, and can be collected by filtration if it is a solid or it can be extracted into an inert organic solvent, such as methylene chloride and the like. Then the solution is dried and the solvent removed.

## Compound VI - Carbamate

The carbamate of formula VI is typically prepared from the 5-aminoisothiazole of formula V and a chloro or bromo phenylformate, in an inert organic solvent, such as toluene. In addition, an acid receptor, such as an inorganic or organic base, should be present to react with the hydrogen chloride formed as a byproduct. The preferred base to be used is pyridine.

The carbamate, Compound VI, can be isolated, if desired, by recrystallization from typical solvents such as ethanol, acetone, and the like, with an ethanol-water mixture being preferred.

Compound VII - Urea

The 3-aryl-5-isothiazolylureas of formula VII can be prepared from the carbamates of formula VI or directly from the 5-aminoisothiazoles of formula V. The preferred method of preparation is using the carbamate, Compound VI, which is dissolved in an inert organic solvent, such as dimethylformamide (DMF), THF, benzene, and the like, with benzene being the preferred solvent. Then the appropriately substituted amine of the formula $HNRR^1$ is added and the entire reaction is run for about 1/2 to about 24 hours at a temperature from about 25 to about 100°C. The preferred reaction conditions are about 80°C for about 1 hour.

A tertiary amine, such as triethylamine, is added when the substituted amine is an amine salt or free methoxymethylamine.

Certain ureas of formula VII, the N-methyl-N-methoxy derivatives of formula VIIA, can be prepared by the alkylation of the N'-(3-substituted-5-isothiazolyl)-N-hydroxyurea, using dimethyl sulfate. The hydroxyurea is generated from the appropriate carbamate of formula VI and hydroxylamine in a process similar to.the process of forming ureas of formula VII from the carbamate of formula VI and substituted amine. Upon formation of the hydroxyurea, the dimethylsulfate is added to form the compounds of formula VIIA.

Another preparation of Compound VII involves the reaction of a 5-aminoisothiazole of formula V with an alkylisocyanate of the formula RNCO. The reaction is

run in an inert organic solvent, such as THF, benzene, ethyl acetate, and the like. Typically dibutyltin diacetate is added to the 5-aminoisothiazole of formula V and then the alkylisocyanate in solvent is added. The reaction is run at a temperature of from about 25 to about 100°C for about 1/2 to about 24 hours.

The 5-aminoisothiazole of formula V can also be reacted with a compound of the formula $COV_2$ wherein V is chloro or bromo, and then reacted with the substituted amine. Typically phosgene is used as the compound of formula $COV_2$ and the isothiazole-phosgene reaction is run in the presence of an acid of the formula HV (with V being chloro or bromo). The reaction is carried out in an inert organic solvent, such as toluene, and the like, at elevated temperatures of from about 60 to about 110°C. The substituted amine is then added (optionally in an inert organic solvent, such as toluene, chloroform, and the like) and the reaction run at a temperature from about 0 to about 100°C.

Another method of preparing the urea of formula VII is from the reaction of the 5-aminoisothiazole of formula V and a substituted cambamoyl halide of the formula, $RR^1NCOV$. The halide is placed in pyridine or in an inert organic solvent, such as DMF, THF, benzene, and the like, with an acid acceptor, such as pyridine, present. Then the isothiazole of formula V is added and the reaction is run for about 1 to about 16 hours at a temperature from about 20 to about 60°C. Preferably, the reaction is at about 25°C for about 2 hours.

The desired urea of formula VII is isolated, if desired, by removing the solvent under vacuum and extracting with dilute acids, such as hydrochloric acid, sulfuric acid, and the like. After the extract is washed, the solution is made basic with sodium bicarbonate, sodium carbonate, sodium hydroxide, and the like. The urea can be recrystallized from typical solvents, such as ethanol-water, hexane, acetone, and the like, with ethanol-water being preferred.

Compound VIII - $R^2$ Alkyl-substituted Ureas

Additionally-substituted ureas of formula VIII are prepared by an alkylation reaction between the unsubstituted urea of formula VII or VIIA and an alkyl halide of the formula $R^{2'}Y$, when $R^{2'}$ is $C_1$-$C_3$ alkyl and wherein Y is bromo, chloro, fluoro, or iodo. The unsubstituted urea of formula VII or VIIA and alkyl halide are usually reacted in an inert organic solvent, such as DMF and the like, in the presence of a base, such as sodium hydride.

The acid addition salts are formed from the reaction of the urea of formula VII, VIIA or VIII in an inert organic solvent, such as ether and the appropriate acid.

The following examples are illustrative of the compounds of formula IX and the intermediates used in their preparation. All temperatures are in degrees Celsius and the melting points are uncorrected.

## Examples of preparation of Compound I

### Intermediate 1

### Ethyl 3,4-dichlorobenzoate

Sixty grams of thionyl chloride and 78.31 g of 3,4-dichlorobenzoic acid were mixed in benzene and heated at reflux temperature until the hydrogen chloride gas evolution stopped. The mixture was cooled and the benzene and excess thionyl chloride removed.

Ethanol was added to form the product, ethyl 3,4-dichlorobenzoate, as an oily precipitate. Yield = 72%.

NMR ($CDCl_3$/DMSO): δ8.1-7.2 (m, 3H); δ4.4 (q, 2H); δ1.45 (t, 3H).

### Intermediate 2

### Ethyl 2,4-dichlorobenzoate

About 200 g of 2,4-dichlorobenzoyl chloride was mixed in 700 ml of ethanol and stirred for about two hours. After the excess ethanol was removed, the yield of the resulting oil was 20%.

NMR ($CDCl_3$): δ7.9-7.7 (d, 1H); δ7.5-7.1 (m, 2H); δ4.0 (q, 2H); δ1.45 (t, 3H).

The following example was prepared using the general procedure of Intermediate 2.

Intermediate 3

Ethyl 4-chlorobenzoate

Yield = 40%.

NMR ($CDCl_3$): δ8.2-7.7 (d, 2H); δ7.5-7.2 (d, 2H); δ4.4 (q, 2H); δ1.4 (t, 3H).

The following examples were prepared using the general procedure of Intermediate 2, except methanol was used as the solvent.

Intermediate 4

Methyl 2,4-dichlorobenzoate

Yield = 80%.

BP (boiling point) = 127-134°/8 mm

Calculated for $C_8H_6Cl_2O_2$:

Theory: C, 46.86; H, 2.95; Cl, 34.58

Found: C, 46.64; H, 2.82; Cl, 34.46.

NMR ($CDCl_3$): δ7.8-7.6 (d, 1H); δ7.4-7.1 (m, 2H); δ3.9 (s, 3H);

Intermediate 5

Methyl 2-chlorobenzoate

Yield = 60%.

BP = 103-125°/8 mm

NMR ($CDCl_3$): δ7.7-6.8 (m, 4H); δ3.6 (s, 3H).

## Examples of preparation of Compound II

### Intermediate 6

### 3-(4-Chlorophenyl)-3-ketopropionitrile

A dispersion of 32 g of 61% sodium hydride in mineral oil was added to 750 ml of tetrahydrofuran (THF). This solution was stirred under reflux as a solution of 73.8 g (0.4 mole) of ethyl (4-chlorophenyl)-acetate and 32.8 g (0.8 mole) of acetonitrile in 150 ml of THF was added dropwise. The refluxing was maintained for about 16 hours.

The solution was cooled and 40 ml of isopropyl alcohol was added dropwise to the solution and then stirred for 30 minutes. The solvent was then removed under vacuum. The solid was slurried in ether and filtered. The solid was added to 600 ml of water and the mineral oil removed by extraction with 600 ml of hexane. The solution was acidified with concentrated hydrochloric acid. The precipitate was filtered, crystallized from ethanol, and air dried. The yield was 48.5 g (67%) and the MP was 132-134°.

Calculated for $C_9H_6ClNO$:

Theory: C, 60.19; H, 3.37; N, 7.80; Cl, 19.74

Found: C, 60.45; H, 3.18; N, 7.90; Cl, 19.52.

NMR ($CDCl_3$/DMSO): δ8.1-7.7 (d, 2H); δ7.6-7.2 (d, 2H); δ4.3 (s, 2H).

The following examples were prepared using the general procedure of Intermediate 6.

Intermediate 7

3-(2-Chlorophenyl)-3-ketopropionitrile

Yield = 72.4 g (80%)
MP = <100°
Calculated for $C_9H_6ClNO$:
Theory: C, 60.19; H, 3.37; N, 7.80; Cl, 19.74
Found: C, 60.44; H, 3.19; N, 7.75; Cl, 19.58.
NMR ($CDCl_3$): δ7.5 (m, 4H); δ4.2 (s, 2H).

Intermediate 8

3-(3,4-Dichlorophenyl)-3-ketopropionitrile

Yield = 12.5 g (58%)
MP = 109-111°
Calculated for $C_9H_5Cl_2NO$:
Theory: C, 50.50; H, 2.35; N, 6.54; Cl, 33.13
Found: C, 50.77; H, 2.12; N, 6.34; Cl, 33.39.

## Examples of Preparation of Compound III

### Intermediate 9

### 3-Amino-3-(4-chlorophenyl)propenenitrile

One hundred-ten grams (0.8 mole) of 4-chlorobenzonitrile, 65.5 g (1.6 moles) of acetonitrile, 36.8 g (1.6 moles) of sodium, and 500 ml of toluene were stirred under reflux for four hours. The mixture was then cooled and stirred for about 16 hours and the formed solid was collected and washed with ether. Six hundred ml of water was added to dissolve the remaining sodium.

The solid was filtered and dried, then it was recrystallized from 600 ml of ether. The product collected was beige crystals and weighed 111.8 g (70% yield). It had a MP of 143-145°.

The following elemental analysis was obtained:
Calculated for $C_9H_7ClN_2$:

Theory: C, 60.52; H, 3.95; N, 15.68; Cl, 19.85

Found: C, 60.32; H, 3.70; N, 15.46; Cl, 19.67.

The following examples are prepared using the general procedure of Intermediate 9.

Intermediate 10

3-Amino-3-phenylpropenenitrile

Yield = 60 g (52%)
MP = 86-87°

Intermediate 11

3-Amino-3-(3-methylphenyl)propenenitrile

Yield = 42.7 g (33%).
MP = 72-74°

Calculated for $C_{10}H_{10}N_2$:
Theory: C, 75.92; H, 6.37; N, 17.71
Found: C, 75.97; H, 6.17; N, 17.64.

Intermediate 12

3-Amino-3-(4-methoxyphenyl)propenenitrile

Yield = 64.28 g (46%)
MP = 118-120°

Calculated for $C_{10}H_{10}N_2O$:
Theory: C, 68.95; H, 5.79; N, 16.08
Found: C, 69.10; H, 6.05; N, 16.11.

Intermediate 13

3-Amino-3-(4-methylphenyl)propenenitrile

Yield = 53.5 g (42%)
MP = 107-111°
Calculated for $C_{10}H_{10}N_2$:

Theory: C, 75.92; H, 6.37; N, 17.71
Found: C, 75.75; H, 6.15; N, 17.54.

Intermediate 14

3-Amino-3-(4-bromophenyl)propenenitrile

Yield = 99.3 g (55%)
MP = 165°
Calculated for $C_9H_7BrN_2$:

Theory: C, 48.46; H, 3.16; N, 12.56; Br, 35.82
Found: C, 48.23; H, 3.03; N, 12.48; Br, 35.99.

Intermediate 15

3-Amino-3-(2-chlorophenyl)propenenitrile

Yield = 27 g (19%)
MP = 105-107°
Calculated for $C_9H_7ClN_2$:

Theory: C, 60.52; H, 3.95; N, 15.68
Found: C, 60.26; H, 3.69; N. 15.56.

## Intermediate 16

3-Amino-3-(3-chlorophenyl)propenenitrile

Yield = 57.1 g (40%)
MP = 85-87°

Calculated for $C_9H_7ClN_2$:

Theory: C, 60.52; H, 3.95; N, 15.68; Cl, 19.85
Found: C, 60.30; H, 4.09; N, 15.45; Cl, 19.63.

## Intermediate 17

3-Amino-3-(4-fluorophenyl)propenenitrile

Yield = 54.4 g (58%)
MP = 105-107°

Calculated for $C_9H_7FN_2$:

Theory: C, 66.66; H, 4.35; N, 17.27; F, 11.72
Found: C, 66.56; H, 4.39; N, 16.99; F, 11.43.

## Intermediate 18

3-Amino-3-(3-fluorophenyl)propenenitrile

Yield = 85.1 g (75%)
MP = 82-84°

Calculated for $C_9H_7FN_2$:

Theory: C, 66.66; H, 4.35; N, 17.27; F, 11.72
Found: C, 66.71; H, 4.14; N, 17.54; F, 11.71.

Intermediate 19

3-Amino-3-(3,5-dimethoxyphenyl)propenenitrile

Yield = 69.2 g (57%)
MP = >114-116°

Calculated for $C_{11}H_{12}N_2O_2$:

Theory: C, 65.01; H, 5.46; N, 13.78
Found: C, 64.76; H, 5.69; N, 14.02.

Intermediate 20

3-Amino-3-(3-bromophenyl)propenenitrile

Yield = 22.35 g (50%)
MP = 95-97°

Calculated for $C_9H_7BrN_2$:

Theory: C, 48.46; H, 3.16; N, 12.56; Br, 35.82
Found: C, 48.59; H, 2.99; N, 12.42; Br, 35.97.

Intermediate 21

3-Amino-3-(3-methoxyphenyl)propenenitrile

Yield = 51.8 g (42%)
MP = 61-63°

Calculated for $C_{10}H_{10}N_2O$:

Theory: C, 68.95; H, 5.79; N, 16.08
Found: C, 68.82; H, 5.87; N, 15.87.

## Intermediate 22

## 3-Amino-3-(4-chlorophenyl)propenenitrile

Forty-eight and one-half grams (0.27 mole) of 3-(4-chlorophenyl)-3-ketopropionitrile was added to 140 ml of liquid ammonia and 350 ml of ethanol. The mixture was heated in a sealed vessel at about 150° for about 16 hours. The excess ammonia and ethanol were removed and then the solid was added to hot ethanol and then cooled. The resulting precipitate was filtered and air dried. The yield was 34.75 g (72%) and the MP was 130-132°.

The following elemental analysis was obtained:

Calculated for $C_9H_7ClN_2$:

Theory: C, 60.52; H, 3.95; N, 15.68; Cl, 19.85

Found: C, 60.46; H, 4.03; N, 15.47; Cl, 20.13.

## Examples of preparation of Compound IV

## Intermediate 23

## 3-Amino-3-(4-chlorophenyl)propenethioamide

Seventy-three grams of 3-amino-3-(4-chlorophenyl)propenenitrile and 500 ml of methylene chloride were added to 62 ml of liquid hydrogen sulfide and 1.0 g of potassium hydroxide. The reaction mixture was kept in a sealed vessel at about 80° for about 24 hours.

A yellow solid was collected and then washed with methylene chloride. This solid had a melting point greater than 245° and softened at about 185°. The methylene chloride was removed from the filtrate under vacuum and then toluene was added to the residue. The resulting yellow solid had a MP of 176-181°. It was recrystallized from ethanol and then had a MP of 180-183°.

The first yellow solid was boiled in 700 ml ethanol and filtered. Then about 250 ml of water was added to the filtrate, the solution was cooled, and a yellow solid was collected with a MP of 178-180°.

Both crops were combined to give 33 g (38% yield).

Calculated for $C_9H_9ClN_2S$:

Theory: C, 50.82; H, 4.27; N, 13.17

Found: C, 50.89; H, 4.25; N, 12.99.

NMR (DMSO-$d_6$): δ8.2 (broad, 2H);; δ7.3 (broad, 2H); δ6.8 (m, 5H); δ3.8 (s, 1H).

The following examples were prepared using the general procedure of Intermediate 23.

Intermediate 24

3-Amino-3-(4-methoxyphenyl)propenethioamide

Yield = 57 g (75%)

MP = 186-189°

Calculated for $C_{10}H_{12}N_2OS$:

Theory; C, 57.67; H, 5.81; N, 13.45

Found: C, 57.94; H, 5.54; N, 13.35.

Intermediate 25

3-Amino-3-phenylpropenethioamide

Yield = 5.5 g (13%)
MP = 171-172°

Calculated for $C_9H_{10}N_2S$:

Theory: C, 60.64; H, 5.65; N, 15.72; S, 17.99

Found: C, 60.37; H, 5.48; N, 15.45; S, 18.00.

Intermediate 26

3-Amino-3-(3-methylphenyl)propenethioamide

Yield = 30 g (57%)
MP = 140-142°

Intermediate 27

3-Amino-3-(4-methylphenyl)propenethioamide

Yield = 40.4 g (69%)
MP = 157-159°

Calculated for $C_{10}H_{12}N_2S$:

Theory: C, 62.46; H, 6.29; N, 14.57; S, 16.68

Found: C, 62.25; H, 6.06; N, 14.31; S, 16.81.

Intermediate 28

3-Amino-3-(3-bromophenyl propenethioamide

Yield = 3.5 g (13%)

Calculated for $C_9H_9BrN_2S$:

Theory: C, 42.04; H, 3.53; N, 10.89; S, 12.47; Br, 31.07

Found: C, 42.05; H, 3.75; N, 10.61; S, 12.39; Br, 31.22.

NMR (DMSO): δ8.9-8.4 (m, 2H); δ8.2-7.0 (m, 6H); δ5.5 (s, 1H).

Intermediate 29

3-Amino-3-(3-chlorophenyl)propenethioamide

Yield = 11 g (19%)

NMR (DMSO): δ8.9-8.5 (m, 2H); δ8.3-7.3 (m, 6H); δ5.5 (s,1H).

Intermediate 30

3-Amino-3-(4-bromophenyl)propenethioamide

Yield = 49 g (63%)

NMR (DMSO): δ8.9-8.5 (m, 2H); δ8.3-7.4 (m, 4H); δ5.5 (s, 1H).

Intermediate 31

3-Amino-3-(4-fluorophenyl)propenethioamide

Yield = 20.8 g (40.7%)
MP = 193-195°

Intermediate 32

3-Amino-3-(3-fluorophenyl)propenethioamide

Yield = 20.25 g (38.2%)
MP = 88-90°

Intermediate 33

3-Amino-3-(3,5-dimethoxyphenyl)propenethioamide

Yield = 36 g (50%)
MP = 146-148°

Intermediate 34

3-Amino-3-(3-methoxyphenyl)propenethioamide

Yield = 13.1 g (24%)

Calculated for $C_{10}H_{12}N_2OS$:

Theory: C, 57.67; H, 5.81; N, 13.45; S, 15.39

Found: C, 57.63; H, 6.02; N, 13.24; S, 15.64.

NMR (DMSO): δ8.9-8.5 (m, 2H); δ8.2-7.7 (m, 2H); δ7.3 (m, 4H); δ5.5 (s, 1H); δ3.8 (s, 3H).

Intermediate 35

3-Amino-3-phenylpropenethioamide

A solution of 21.2 g (0.47 mole) of 3-phenyl-3-aminopropenenitrile, 0.54 g (0.0025 mole) of sodium sulfide nonahydrate, 1.0 g (0.0025 mole) tetrabutylammonium chloride, 50 ml of benzene, and 25 ml of water was stirred at about 70° under an atmosphere of hydrogen sulfide for about 16 hours.

The solution was cooled over the weekend, forming a precipitate. It was filtered and crystallized from ethanol and then air dried. The yield was 3.9 g (15%). MP = 155-165°C.

NMR ($DMSO-d_6$): δ8.9 (broad, 2H); δ8.0 (broad, 2H); δ7.5 (m, 5H); δ5.5 (s, 1H).

Examples of preparation of Compound V

Intermediate 36

5-Amino-3-(4-chlorophenyl)isothiazole

A solution of 21.3 g (0.1 mole) of 3-amino-3-(4-chlorophenyl)propenethioamide, 29.2 g (0.21 mole) of potassium carbonate, and 1,000 ml of ether was stirred under reflux. Then 25.4 g (0.1 mole) of iodine in 250 ml of ether was added dropwise to the solution.

Then it was stirred under reflux for 4 more hours and cooled. Five hundred ml of water was added, the resulting layers were separated, and the ether layer was dried over magnesium sulfate.

The ether was removed under vacuum leaving a yellow solid, which was then recrystallized from toluene. The MP was 128-129° and the yield was 17 g (81%).

The following elemental analysis was obtained:

Calculated for $C_9H_7ClN_2S$:

Theory: C, 51.33; H, 3.35; N, 13.30

Found: C, 51.54; H, 3.10; N, 13.26.

NMR (DMSO-$d_6$): δ7.95 (d, 2H); δ7.55 (d, 2H); δ6.8 (broad, 2H); δ6.75 (s, 1H).

The following examples were prepared using the general procedure of Intermediate 36.

## Intermediate 37

### 5-Amino-3-phenylisothiazole

Yield = 2.5 g (52%)

MP = 161-163°

Calculated for $C_9H_8N_2S$:

Theory: C, 61.34; H, 4.58; N, 15.90

Found: C, 61.37; H, 4.30; N, 15.67.

NMR (DMSO-$d_6$): δ7.9 (m, 2H); δ7.4 (m, 3H); δ6.7 (s, 1H); δ6.4 (broad, 2H).

Intermediate 38

5-Amino-3-(4-methoxyphenyl)isothiazole

Yield = 7 g (20%)

MP = 188-190°

Calculated for $C_{10}H_{10}N_2OS$:

Theory: C, 58.23; H, 4.89; N, 13.58; S, 15.54

Found: C, 58.40; H, 4.85; N, 13.36; S, 15.70.

Intermediate 39

5-Amino-3-(4-methylphenyl)isothiazole

Yield = 19 g (58%)

MP = 122-124°

Calculated for $C_{10}H_{10}N_2S$:

Theory: C, 63.13; H, 5.30; N, 14.72; S, 16.85

Found: C, 63.01; H, 5.31; N, 14.71; S, 16.61.

NMR (DMSO): δ7.7 (d, 2H); δ7.1 (d, 2H); δ6.6 (s, 1H); δ2.3 (s, 3H).

## Intermediate 40

### 5-Amino-3-(3-methylphenyl)isothiazole

Yield = 71%
MP = 78-80°

Calculated for $C_{10}H_{10}N_2S$:

Theory: C, 63.13; H, 5.30; N, 14.72; S, 16.85

Found: C, 62.54; H, 4.99; N, 13.86; S, 16.02.

## Intermediate 41

### 5-Amino-3-(3-chlorophenyl)isothiazole

Yield = 17 g (62%)
MP = 106-107°

Calculated for $C_9H_7ClN_2S$:

Theory: C, 51.31; H, 3.35; N, 13.30; S, 15.22; Cl, 16.83

Found: C, 51.57; H, 3.57; N, 13.05; S, 14.99; Cl, 16.65.

NMR ($CDCl_3$/DMSO): δ7.7 (m, 3H); δ7.3 (d, 1H); δ6.6 (s, 1H); δ5.5 (broad, 2H).

Intermediate 42

5-Amino-3-(4-fluorophenyl)isothiazole

Yield = 9.7 g (50%)

Calculated for $C_9H_7FN_2S$:

Theory: C, 55.65; H, 3.63; N, 14.42; S, 16.51; F, 9.78

Found: C, 55.73; H, 3.55; N, 14.15; S, 16.21; F, 9.84.

NMR (DMSO): δ8.0 (q, 2H); δ7.2 (t, 2H); δ6.7 (s, 1H); δ6.6 (broad, 2H).

Intermediate 43

5-Amino-3-(3,5-dimethoxyphenyl)isothiazole

Yield = 29.2 g (98%)

NMR (DMSO-$d_6$): δ7.0 (s, 2H); δ6.8 (s, 1H); δ6.6 (s, 1H); δ6.2 (broad, 2H); δ3.9 (s, 6H).

Intermediate 44

5-Amino-3-(3-methoxyphenyl)isothiazole

Yield = 9.9 g (82%)

NMR ($CDCl_3$/DMSO-$d_6$): δ7.25 (t, 3H); δ6.8 (m, 1H); δ5.6 (broad, 2H); δ3.8 (s, 3H).

## Intermediate 45

## 5-Amino-3-(3-fluorophenyl)isothiazole

Yield = 7 g (89%)

NMR ($CDCl_3$/DMSO-$d_6$): δ7.8-7.2 (m, 4H); δ6.6 (s, 1H); δ6.2 (broad, 2H).

## Examples of preparation of Compound VI

## Intermediate 46

## Phenyl N-(3-phenyl-5-isothiazolyl)carbamate

Two grams (0.011 mole) of 5-amino-3-phenylisothiazole was stirred in 15 ml of pyridine. Then 1.8 g (0.011 mole) of phenylchloroformate was added dropwise. The solution was stirred for 1.5 hours, then 80 ml of ice-water was added, and a solid precipitate was collected.

The solid was recrystallized from ethyl acetate and had a MP of 214-216°. The yield was 2.8 g (86%).

The following elemental analysis was obtained:

Calculated for $C_{16}H_{12}N_2O_2S$:

Theory: C, 64.85; H, 4.08; N, 9.45

Found: C, 64.60; H, 4.07; N, 9.41.

The following examples were prepared using the general procedure of Intermediate 46.

## Intermediate 47

Phenyl N-[3-(4-chlorophenyl)-5-isothiazolyl]carbamate

Yield = 14.3 g (75%)

MP = 261-265°

Calculated for $C_{16}H_{11}ClN_2O_2S$:

Theory: C, 58.10; H, 3.35; N, 8.47

Found: C, 57.97; H, 3.49; N, 8.47.

## Intermediate 48

Phenyl N-[3-(4-methoxyphenyl)-5-isothiazolyl]carbamate

Yield = 16.3 g (63%)

MP = 230-232°

Calculated for $C_{17}H_{14}N_2O_3S$:

Theory: C, 62.56; H, 4.32; N, 8.58; S, 9.82

Found: C, 62.75; H, 4.31; N, 8.59; S, 9.60.

## Intermediate 49

Phenyl N-[3-(4-methylphenyl)-5-isothiazolyl]carbamate

Yield = 8.75 g (28%)

MP = 239-241°

Calculated for $C_{17}H_{14}N_2O_2S$:

Theory: C, 65.79; H, 4.55; N, 9.03; S, 10.33

Found: C, 65.65; H, 4.47; N. 8.78; S, 10.20.

Intermediate 50

Phenyl N-[3-(3-methylphenyl)-5-isothiazolyl]carbamate

Yield = 30 g (97%)
MP = 155-157°

Intermediate 51

Phenyl N-[3-(4-fluorophenyl)-5-isothiazolyl]carbamate

Yield = 11.45 g (73%)

Calculated for $C_{16}H_{11}FN_2O_2S$:

Theory: C, 61.14; H, 3.53; N, 8.91; S, 10.20; F, 6.04

Found: C, 61.12; H, 3.33; N, 9.17; S, 10.17; F, 6.17.

NMR (DMSO-$d_6$): δ12.2 (broad, 1H); δ8.0 (q, 2H); δ7.3 (m, 8H).

Intermediate 52

Phenyl N-[3-(4-bromophenyl)-5-isothiazolyl]carbamate

Yield = 40 g (71%)
MP = 208-210°

Intermediate 53

Phenyl N-[3-(3-methoxyphenyl)-5-isothiazolyl]carbamate

Yield = 5.9 g (91%)

Calculated for $C_{17}H_{14}N_2O_3S$:

Theory: C, 62.56; H, 4.32; N, 8.58; S, 9.22

Found: C, 62.66; H, 4.44; N, 8.28; S, 9.51.

NMR (DMSO-$d_6$): δ12.2 (broad, 1H); δ7.2 (m, 9H); δ3.8 (s, 3H).

Intermediate 54

Phenyl N-[3-(3-fluorophenyl)-5-isothiazolyl]carbamate

Yield = 9.2 g (79%)

MP = 152-155°

Examples of Preparation of Compounds VII and VIII

Example 1

N'-(3-Phenyl-5-isothiazolyl)-N,N-dimethylurea

A solution of 2.1 g (7.1 mmole) of phenyl N-[3-phenyl-5-isothiazolyl]carbamate, 1.15 g (14 mmole) of dimethylamine hydrochloride, 1.43 g (14 mmole) of triethylamine, and 45 ml of benzene was stirred under reflux for about 1 hour. The solution was cooled, water and hexane were added, and a solid collected.

This solid was recrystallized from ethyl acetate and had a MP of 223-240°. NMR indicated an impurity was present, so the solid was recrystallized from an ethanol-water mixture. The MP was 253-255° and the yield was 1.5 g (86%). NMR then confirmed the proposed structure.

The following elemental analysis was obtained:
Calculated for $C_{12}H_{13}N_3OS$:

Theory: C, 58.28; H, 5.30; N, 16.99
Found: C, 58.14; H, 5.08; N, 16.73.

The following examples were prepared using the general procedure of Example 1, with the appropriate substituted phenyl carbamate and the indicated amine reactant.

## Example 2

N'-[3-(4-Methoxyphenyl)-5-isothiazolyl]-N,N-dimethylurea

Reactant-dimethylamine hydrochloride
Yield = 800 mg (61%)
MP = 263-265°

Calculated for $C_{13}H_{15}N_3O_2S$:

Theory: C, 56.30; H, 5.45; N, 15.15; S, 11.58
Found: C, 56.57; H, 5.60; N, 14.86; S, 11.36.

Example 3

N'-[3-(4-Methoxyphenyl)-5-isothiazolyl]-N-ethyl-N-methylurea

Reactant-methylethylamine
Yield = 0.9 g (60%)
MP = 226-228°

Calculated for $C_{14}H_{17}N_3O_2S$:

Theory: C, 57.71; H, 5.88; N, 14.42; S, 11.00

Found: C, 57.80; H, 5.66; N, 14.47; S, 10.80.

Example 4

N'-[3-(4-Chlorophenyl)-5-isothiazolyl]-N-ethyl-N-methylurea

Reactant-methylethylamine
Yield = 500 mg (56%)
MP = 222-224°

Calculated for $C_{13}H_{14}ClN_3OS$:

Theory: C, 52.79; H, 4.77; N, 14.21

Found: C, 52.90; H, 4.88; N, 14.00.

## Example 5

N'-[3-(4-Methylphenyl)-5-isothiazolyl]-N-ethyl-N-methylurea

Reactant-methylethylamine
Yield = 1.1 g (80%)
MP = 190-192°
NMR (DMSO-$d_6$): δ7.8 (d, 2H); δ7.3 (s, 1H); δ7.2 (d, 2H); δ3.4 (q, 2H); δ3.0 (s, 3H); δ2.3 (s, 3H); δ1.1 (t, 3H).

Calculated for $C_{14}H_{17}N_3OS$:

Theory: C, 61.06; H, 6.22; N, 15.26; S, 11.64

Found: C, 60.79; H, 6.04; N, 15.41; S, 11.76.

## Example 6

N'-[3-(4-Chlorophenyl)isothiazolyl]-N-(1-methylethyl)-N-methylurea

Reactant-methylisopropylamine
Yield = 0.6 g (40%)
MP = 185-187°

Calculated for $C_{14}H_{16}ClN_3OS$:

Theory: C, 54.27; H, 5.21; N, 13.56, S, 10.35; Cl, 11.44

Found: C, 54.25; H, 5.11; N, 13.26; S, 10.18; Cl, 11.20.

Example 7

N'-[3-(4-Methoxyphenyl)-5-isothiazolyl]-N-(2-propenyl)-N-methylurea

Reactant-methyl-2-propenylamine
Yield = 0.6 g (40%)
MP = 188-190°

Calculated for $C_{15}H_{17}N_3O_2S$:
Theory: C, 59.39; H, 5.65; N, 13.85; S, 10.57
Found: C, 59.51; H, 5.53; N, 13.83; S, 10.71.

Example 8

N'-[3-(4-Methylphenyl)-5-isothiazolyl]-N-(2-propenyl)-N-methylurea

Reactant-methyl-2-propenylamine
Yield = 1.1 g (78%)
MP = 170-172°

Calculated for $C_{15}H_{17}N_3OS$:
Theory: C, 62.72; H, 5.92; N, 14.63; S, 11.15
Found: C, 63.10; H, 6.11; N, 14.81; S, 10.71.

## Example 9

N'-[3-(4-Fluorophenyl)-5-isothiazolyl]-N-(2-propenyl)-N-methylurea

Reactant-methyl-2-propenylamine
Yield = 900 mg (64%)
MP = 180-181°

Calculated for $C_{14}H_{14}FN_3OS$:

Theory: C, 57.72; H, 4.84; N, 14.42; S, 11.01; F, 6.52

Found: C, 57.93; H, 5.03; N, 14.41; S, 10.88; F, 6.72.

## Example 10

N'-[3-(4-Fluorophenyl)-5-isothiazolyl]-N-(1-methylethyl)-N-methylurea

Reactant-methylisopropylamine
Yield = 2.1 g (72%)
MP = 190-193°

Calculated for $C_{14}H_{16}FN_3OS$:

Theory: C, 57.32; H, 5.50; N, 14.32; S, 10.93; F, 6.48

Found: C, 57.59; H, 5.65; N, 14.21; S, 10.72; F, 6.43.

## Example 11

N'-[3-(4-Chlorophenyl)-5-isothiazolyl]-N-(1-methylpropyl)-N-methylurea

Reactant-methylsecbutylamine
Yield = 0.6 g (75%)
MP = 191-193°
Calculated for $C_{15}H_{18}ClN_3OS$:
Theory: C, 55.63; H, 5.60; N, 12.98;
Found: C, 55.88; H, 5.59; N, 13.06.

## Example 12

N'-[3-(4-Chlorophenyl)-5-isothiazolyl]-N-butyl-N-methylurea

Reactant-methylbutylamine
Yield = 1.65 g (26%)
MP = 170-173°
Calculated for $C_{15}H_{18}ClN_3OS$:
Theory: C, 55.63; H, 5.60; N, 12.98; S, 9.90; Cl, 10.95
Found: C, 56.00; H, 5.81; N, 13.01; S, 10.05; Cl, 11.02.

Example 13

N'-[3-(4-Fluorophenyl)-5-isothiazolyl]-N-ethyl-N-methylurea

Reactant-methylethylamine
Yield = 3.5 g (64%)
MP = 162-165°

Calculated for $C_{13}H_{14}FN_3OS$:

Theory: C, 55.90; H, 5.05; N, 15.04; S, 11.48; F, 6.80

Found: C, 55.95; H, 5.20; N, 14.81; S, 11.44; F, 7.75 and 8.06.

Example 14

N'-(3-Phenyl-5-isothiazolyl)-N-(1-methylethyl)-N-methylurea

Reactant-methylisopropylamine
Yield = 1.8 g (66%)
MP = 230-233°

Calculated for $C_{14}H_{17}N_3OS$:

Theory: C, 61.06; H, 6.22; N, 15.29; S, 11.64

Found: C, 60.81; H, 5.83; N, 14.90; S, 11.42.

Example 15

N'-[3-(3-Methylphenyl)-5-isothiazolyl]-N-(1-methylethyl)-N-methylurea

Reactant-methylisopropylamine
Yield = 1.7 g (59%)
MP = 205-207°

Calculated for $C_{15}H_{19}N_3OS$:

Theory: C, 62.25; H, 6.62; N, 14.52; S, 11.08
Found: C, 62.48; H, 6.46; N, 14.25; S, 10.82.

Example 16

N'-[3-(3-Methylphenyl)-5-isothiazolyl]-N-ethyl-N-methylurea

Reactant-methylethylamine
Yield = 4.65 g (85%)
MP = 119-122°

Calculated for $C_{14}H_{17}N_3OS$:

Theory: C, 61.06; H, 6.22; N, 15.26; S, 11.64
Found: C, 61.28; H, 6.33; N, 14.99; S, 11.39.

Example 17

N'-[3-(4-Chlorophenyl)-5-isothiazolyl]-N-methoxy-N-methylurea

Reactant-methoxymethylamine
Yield = 1.7 g (57%)
MP = 207-209°
Calculated for $C_{12}H_{12}ClN_3O_2S$:
Theory: C, 48.40; H, 4.06; N, 14.11; S, 10.77; Cl, 11.91
Found: C, 48.65; H, 3.92; N, 14.18; S, 10.49; Cl, 12.02.

Example 18

N'-[3-(4-Methoxyphenyl)-5-isothiazolyl]-N-methoxy-N-methylurea

Reactant-methoxymethylamine
Yield = 1 g (68%)
MP = 210-212°
Calculated for $C_{13}H_{15}N_3O_3S$:
Theory: C, 53.23; H, 5.15; N, 14.32; S, 10.93
Found: C, 53.43; H, 5.04; N, 14.14; S, 10.65.

Example 19

N'-[3-(4-Methylphenyl)-5-isothiazolyl]-N,N-dimethylurea

Reactant-dimethylamine hydrochloride
Yield = 0.4 g (30%)
MP = 269-271°

Calculated for $C_{13}H_{15}N_3OS$:

Theory: C, 59.75; H, 5.79; N, 16.08; S, 12.27

Found: C, 59.72; H, 5.55; N, 15.88; S, 12.26.

Example 20

N'-[3-(3-Methylphenyl)-5-isothiazolyl]-N,N-dimethylurea

Reactant-dimethylamine hydrochloride
Yield = 1.5 g (57%)
MP = 206-208°

Calculated for $C_{13}H_{15}N_3OS$:

Theory: C, 59.75; H, 5.79; N, 16.08; S, 12.27

Found: C, 59.89; H, 5.65; N, 16.10; S, 11.95.

## Example 21

N'-[3-(3-Chlorophenyl)-5-isothiazolyl]-N,N-dimethylurea

Reactant-dimethylamine hydrochloride

Yield = 1 g (64%)

MP = 130-132°

Calculated for $C_{12}H_{12}ClN_3OS$:

Theory: C, 51.15; H, 4.29; N, 14.91; S, 11.38; Cl, 12.58

Found: C, 51.40; H, 4.39; N, 14.63; S, 11.16; Cl, 12.73.

## Example 22

N'-[3-(3-Chlorophenyl)-5-isothiazolyl]-N-methoxy-N-methylurea

Reactant-methoxymethylamine hydrochloride

Yield = 1 g (68%)

Calculated for $C_{12}H_{12}ClN_3O_2S$:

Theory: C, 48.40; H, 4.06; N, 14.11; S, 10.77; Cl, 11.91

Found: C, 48.38; H, 3.92; N, 14.28; S, 10.72; Cl, 12.02.

NMR (DMSO-$d_6$): δ11.2 (broad, 1H); δ7.9 (m, 2H); δ7.4 (d, 2H); δ3.7 (s, 3H); δ3.2 (s, 3H).

## Example 23

N'-[3-(4-Methylphenyl)-5-isothiazolyl]-N-methoxy-N-methylurea

Reactant-methoxymethylamine hydrochloride
Yield = 1 g (77%)
MP = >260°

Calculated for $C_{13}H_{15}N_3O_2S$:

Theory: C, 56.30; H, 5.45; N, 15.15; S, 11.56

Found: C, 56.37; H, 5.20; N, 15.40; S, 11.33.

NMR (DMSO-$d_6$): δ11.2 (broad, 1H); δ7.8 (d, 2H); δ7.3 (t, 3H); δ3.7 (s, 3H); δ3.2 (s, 3H); δ2.3 (s, 3H).

## Example 24

N'-[3-(4-Fluorophenyl)-5-isothiazolyl]-N,N-dimethylurea

Reactant-dimethylamine hydrochloride
Yield = 84%
MP = 235-237°

Calculated for $C_{12}H_{12}FN_3OS$:

Theory: C, 54.33; H, 4.56; N, 15.84; S, 12.09; F, 7.16

Found: C, 54.53; H, 4.35; N, 16.05; S, 12.26; F, 6.88.

Example 25

N'-[3-(3-Methylphenyl)-5-isothiazolyl]-N-methoxy-N-methylurea

Reactant-methoxymethylamine hydrochloride
Yield = 700 mg (26%)
MP = 99-101°

Calculated for $C_{13}H_{15}N_3O_2S$:

Theory: C, 56.30; H, 5.45; N, 5.15; S, 11.56

Found: C, 56.58; H, 5.41; N, 4.97; S, 11.45.

Example 26

N'-[3-(4-Bromophenyl)-5-isothiazolyl]-N,N-dimethylurea

Reactant-dimethylamine hydrochloride
Yield = 300 mg (18%)
MP = 240-242°

Calculated for $C_{12}H_{12}BrN_3OS$:

Theory: C,44.18; H, 3.71; N, 12.88; S, 9.83; Br, 24.49

Found: C, 43.89; H, 3.58; N, 12.60; S, 9.63; Br, 24.69.

## Example 27

N'-[3-(3-Methoxyphenyl)-5-isothiazolyl]-N,N-dimethylurea

Reactant-dimethylamine hydrochloride
Yield = 0.98 g (75%)
MP = 153-155°

Calculated for $C_{13}H_{15}N_3O_2S$:

Theory: C, 56.30; H, 5.45; N, 15.15; S, 11.56

Found: C, 56.04; H, 5.17; N, 14.86; S, 11.35.

## Example 28

N'-(3-Phenyl-5-isothiazolyl)-N-methoxy-N-methylurea

Reactant-methoxymethylamine hydrochloride
Yield = 1.6 g (32%)
MP = 154-156°

Calculated for $C_{12}H_{13}N_3O_2S$:

Theory: C, 54.74; H, 4.98; N, 15.96; S, 12.18

Found: C, 54.93; H, 4.68; N, 15.69; S, 12.00.

## Example 29

N'-[3-(4-Fluorophenyl)-5-isothiazolyl]-N-(2-methypropyl)-N-methylurea

Reactant-methyl-2-methylpropylamine
Yield = 1.5 g (49%)
MP = 140-142°

Calculated for $C_{15}H_{18}FN_3OS$:

Theory: C, 58.61; H, 5.90; N, 13.67; S, 10.43; F, 6.18

Found: C, 58.67; H, 5.95; N, 13.50; S, 10.48; F 6.33.

## Example 30

N'-[3-(4-Fluorophenyl)-5-isothiazolyl]-N-n-butyl-N-methylurea

Reactant-n-butylmethylamine
Yield = 2 g (65%)
MP = 174-176°

Calculated for $C_{15}H_{18}FN_3OS$:

Theory: C, 58.61; H, 5.90; N, 13.67; S, 10.43; F, 6.18

Found: C, 58.39; H, 5.67; N, 13.64; S, 10.17; F, 6.28.

Example 31

N'-[3-(4-Fluorophenyl)-5-isothiazolyl]-N-methyl-N-pentylurea

Reactant-methylpentylamine
Yield = 2g (62%)
MP = 184-186°

Calculated for $C_{16}H_{20}FN_3OS$:

Theory: C, 59.79; H, 6.27; N, 13.07; S, 9.98; F, 5.91

Found: C, 60.02; H, 6.53; N, 13.01; S, 9.93; F, 6.01.

Example 32

N'-[3-(4-Fluorophenyl)-5-isothiazolyl]-N-methoxy-N-methylurea

Reactant-methoxymethylamine hydrochloride
Yield = 3.0 g (52%)
MP = 137-139°

Calculated for $C_{12}H_{12}FN_3O_2S$:

Theory: C, 51.24; H, 4.30; N, 14.94

Found: C, 51.45; H, 4.46; N, 15.09.

Example 33

N'-[3-(4-Fluorophenyl)-5-isothiazolyl]-N-(2-butyl)-N-methylurea

Reactant-2-butylmethylamine
Yield = 6.65 g (72%)
MP = 140°
Calculated for $C_{15}H_{18}FN_3OS$:
Theory: C, 58.61; H, 5.90; N, 13.67
Found: C, 58.34; H, 5.63; N, 13.46.

Example 34

N'-[3-(4-Fluorophenyl)-5-isothiazolyl]-N-(1,1-dimethylethyl)-N-methylurea

Reactant-(tert-butyl)methylamine
Yield = 4.7 g (77%)
MP = 200-203°
Calculated for $C_{15}H_{18}FN_3OS$:
Theory: C, 58.61; H, 5.90; N, 13.67
Found: C, 58.82; H, 5.92; N, 13.47.

Example 35

N'-[3-(4-Chlorophenyl)-5-isothiazolyl]-N,N-dimethylurea

Under nitrogen, a solution of 2.1 g of 5-amino-3-(4-chlorophenyl)isothiazole in 20 ml of dimethylformamide was added slowly to 0.5 g of a 57%

dispersion of sodium hydride in mineral oil also in dimethylformamide. After 15 minutes, 1.1 g of N,N-dimethylcarbamyl chloride was added. The resulting solution was stirred for about 16 hours with heating to about 40°.

Once the heat was removed, the solution was poured into water. The resulting precipitate was recrystallized from ethyl acetate and had a MP of 261-267°. Yield = 1 g (36%).

The following elemental analysis was obtained:
Calculated for $C_{12}H_{11}ClN_3OS$:

Theory: C, 51.15; H, 4.29; N, 14.91
Found: C, 51.00; H, 4.34; N, 15.13.

## Example 36

N'-[3-(4-Chlorophenyl)-5-isothiazolyl]-N',N,N-trimethylurea

A solution of 0.64 g (0.016 mole) of 60% sodium hydride in mineral oil was washed with hexane. Then 15 ml of dimethylformamide was added with stirring, then 4.0 g (0.014 mole) of N'-[3-(4-chlorophenyl)-5-isothiazolyl]-N,N-dimethylurea was added in portions. The solution was stirred 15 minutes, cooled in an ice bath, and then 2.8 g (0.02 mole) of methyl iodide was added. It was allowed to warm to room temperature and then poured into ice water.

A white solid was collected and washed with water; it had a MP of 166-169°. Then it was recrystallized from ethyl acetate and had a MP of 172-174°. The yield was 2.9 g (70%). The following elemental analysis was obtained:

Calculated for $C_{13}H_{14}ClN_3OS$:

Theory: C, 52.79; H, 4.77; N, 14.21

Found: C, 52.56; H, 4.82; N, 14.02.

NMR (DMSO-$d_6$): δ7.95 (d, 2H); δ7.45 (d, 2H); δ7.35 (s, 1H); δ3.4 (s, 3H); δ2.8 (s, 6H).

The following examples were prepared using the general procedure of Example 36, except the appropriate substituted iodide was used, along with the indicated urea.

Example 37

N'-[3-(4-Fluorophenyl)-5-isothiazolyl]-N'-ethyl-N,N-dimethylurea

Reactant-N'-[3-(4-fluorophenyl)-5-isothiazolyl]-N,N-dimethylurea

Yield = 1.5 g (51%)

MP = 100-102°

Calculated for $C_{14}H_{16}FN_3OS$:

Theory: C, 57.32; H, 5.50; N, 14.32; S, 10.93; F, 6.48

Found: C, 57.59; H, 5.46; N, 14.53; S, 11.21; F, 6.52.

Example 38

N'-[3-(4-Fluorophenyl)-5-isothiazolyl]-N',N,N-trimethylurea

Reactant-N'-[3-(4-fluorophenyl)-5-isothiazolyl]-N,N-dimethylurea

Yield = 525 mg (18%)

MP = 151-153°

Calculated for $C_{13}H_{14}FN_3OS$:

Theory: C, 55.90; H, 5.05; N, 15.04

Found: C, 56.17; H, 4.80; N, 14.87.

Example 39

N'-[3-(4-Fluorophenyl)-5-isothiazolyl]-N'-ethyl-N-(1-methylethyl)-N-methylurea

Reactant-N'-[3-(4-fluorophenyl)-5-isothiazolyl]-N-(1-methylethyl)-N-methylurea

Yield = 0.35 g (21%)

MP = 70-72°

Calculated for $C_{16}H_{20}FN_3OS$:

Theory: C, 59.79; H, 6.27; N, 13.07; S, 9.98; F, 5.91

Found: C, 59.54; H, 6.04; N, 12.98; S, 10.12; F, 6.16.

Example 40

N'-[3-(4-Chlorophenyl)-5-isothiazolyl]-N'-ethyl-N,N-dimethylurea

Reactant-N'-[3-(4-chlorophenyl)-5-isothiazolyl]-N,N-dimethylurea

Yield = 1 g (16%)

MP = 116-118°

Calculated for $C_{14}H_{16}ClN_3OS$:

Theory: C, 54.27; H, 5.21; N, 13.56; S, 10.35; Cl, 11.44

Found: C, 54.37; H, 5.33; N, 13.32; S, 10.09; Cl, 11.86.

Example 41

N'-[3-(4-Chlorophenyl)-5-isothiazolyl]-N'-ethyl-N-(1-methylethyl)-N-methylurea

Reactant-N'-[3-(4-chlorophenyl)-5-isothiazolyl]-N-(1-methylethyl)-N-methylurea

Yield = 1.5 g (44%)

MP = 105-107°

Calculated for $C_{16}H_{20}ClN_3OS$:

Theory: C, 56.88; H, 5.97; N, 12.44; S, 9.49; Cl, 10.49

ınd: C, 56.89; H, 6.15; N, 12.61; S, 9.38; Cl, 10.43.

Example 42

N'-[3-(4-Fluorophenyl)-5-isothiazolyl]-N',N-dimethyl-N-(1-methylethyl)urea

Reactant-N'-[3-(4-fluorophenyl)-5-isothiazolyl]-N-(1-methylethyl)-N-methylurea

Yield = 3 g (65%)

MP = 162-164°

Calculated for $C_{15}H_{18}FN_3OS$:

Theory: C, 58.61; H, 5 90; N, 13.67; S, 10.43

Found: C, 58.86; H, 5.79; N, 13.57; S, 10.61.

Example 43

N'-[3-(4-Fluorophenyl)-5-isothiazolyl]-N'-ethyl-N-methoxy-N-methylurea

Reactant-N'-[3-(4-fluorophenyl)-5-isothiazolyl]-N-methoxy-N-methylurea

Yield = 1.5 g (46%)

MP = 115-117°

Calculated for $C_{14}H_{16}FN_3O_2S$:

Theory: C, 54.36; H, 5.21; N, 13.58

Found: C, 54.08; H, 5.40; N, 13.33.

## Example 44

N'-[3-(4-Fluorophenyl)-5-isothiazolyl]-N-methoxy-N-methylurea monohydrochloride

Six grams (0.02 mole) of phenyl N-[3-(4-fluorophenyl)-5-isothiazolyl]carbamate, 6.83 g (0.07 mole) of methoxymethylamine hydrochloride, and 7.07 g (0.07 mole) of triethylamine were mixed in 40 ml of benzene and the resulting solution was warmed at 75° for about 16 hours.

The solution was cooled, 200 ml of ether was added, and then the solution was washed with water. After two washes, 150 ml of hydrochloric acid was added. A precipitate formed in the hydrochloric acid and at the interface. This precipitate was filtered off and crystallized from ethanol ether. The yield was 3 g (47%) and the MP was 194-197° with decomposition.

The following elemental analysis was obtained:

Calculated for $C_{12}H_{12}FN_3O_2S \cdot HCl$:

Theory: C, 45.36; H, 4.12; N, 13.22; S, 10.09

Found: C, 45.68; H, 3.85; N, 13.43; S, 10.18.

The following examples were prepared using the general procedure of Example 44.

## Example 45

N'-[3-(4-Chlorophenyl)-5-isothiazolyl]-N-(1-methylpropyl)-N-methylurea monohydrochloride

Yield = 325 mg (20%)
MP = 199-205°

Calculated for $C_{15}H_{18}ClN_3OS \cdot HCl$:

Theory: C, 50.00; H, 5.32; N, 11.66; S, 8.90, Cl, 19.68

Found: C, 50.19; H, 5.46; N, 11.91; S, 8.78; Cl, 19.47.

## Example 46

N'-[3-(3-Fluorophenyl)-5-isothiazolyl]-N-methyl-N-(1-methylethyl)urea monohydrochloride

Yield = 0.2 g (14%)
MP = 203-205°

Calculated for $C_{14}H_{16}FN_3OS \cdot HCl$:

Theory: C, 57.32; H, 5.50; N, 14.32; S, 10.93

Found: C, 57.50; H, 5.65; N, 14.41; S, 11.01.

## Algicidal Method

Furthermore, a method is provided for controlling the growth of aquatic algae which comprises

contacting the algae to be controlled or the water in which the algae is growing with an algicidally-effective amount of a compound of the formula

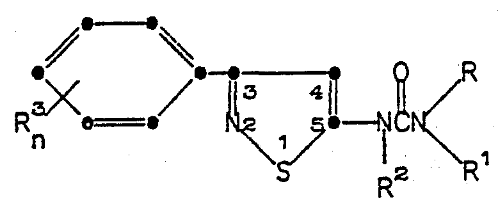

IXA

wherein

R and $R^1$ are $C_1$-$C_6$ alkyl, $C_3$-$C_6$ alkenyl, or $C_1$-$C_2$ alkoxy, provided that one of R or $R^1$ is methyl;

$R^2$ is hydrogen or $C_1$-$C_3$ alkyl;

$R^3$ is hydrogen, halo, $C_1$-$C_2$ alkoxy, or $C_1$-$C_6$ alkyl; and

n is 1 or 2;

or an aquatically-acceptable acid-addition salt thereof, provided that when one of R and $R^1$ is methyl, and the other is 1-methylethyl, and $R^2$ is hydrogen, $R^3$ cannot be 4-fluoro. This means that the compound cannot be N'-[3-(4-fluorophenyl)-5-isothiazolyl]-N-methyl-N-(1-methylethyl)urea.

More preferred compounds of formula IXA within this method are those in which:

R and $R^1$ are $C_1$-$C_4$ alkyl or $C_1$-$C_2$ alkoxy, provided that one of R or $R^1$ is methyl;

$R^2$ is hydrogen;

$R^3$ is hydrogen, halo or methyl; and

n is 1.

Even more preferred compounds of formula IXA are where:

R is $C_1$-$C_4$ alkyl; $R^1$ is methoxy or methyl; provided that one of R or $R^1$ is methyl; and

$R^3$ is 4-fluoro

The representative compounds of formula IXA are:

N'-[3-(4-chlorophenyl)-5-isothiazolyl]-N-methyl-N-(1-methylethyl)urea;

N'-[3-(4-fluorophenyl)-5-isothiazolyl]-N,N-dimethylurea;

N'-[3-(4-bromophenyl)-5-isothiazolyl]-N,N-dimethylurea;

N'-[3-(4-chlorophenyl)-5-isothiazolyl]-N,N-dimethylurea;

N'-(3-phenyl-5-isothiazolyl)-N,N-dimethylurea;

N'-[3-(4-methylphenyl)-5-isothiazolyl]-N,N-dimethylurea;

N'-[3-(4-fluorophenyl)-5-isothiazolyl]-N-ethyl-N-methylurea;

N'-[3-(4-fluorophenyl)-5-isothiazolyl]-N-methoxy-N-methylurea; and

N'-(3-phenyl-5-isothiazolyl)-N-methoxy-N-methylurea.

The compounds of formula IXA have been evaluated in a standard aquatic algicide test designed to show algicidal activity. In a primary screen, the compounds were evaluated against <u>Chlorella vulgaris</u>, <u>Scenedesmus quadricauda</u>, <u>Anacystis nidulans</u>, <u>Chlamydomonas</u>

moewusii, Stichococcus bascillaris, Anabaena flos-aquae, Anabaena spiroides (blue-green), and Anabaena sp. (1551). These algae species were grown on agar slants containing artificial media (Hughes' media). The agar slants were employed in the inoculation of the test media, which itself is an aqueous Hughes' media. Five milliliters of sterile Hughes' media was used to wash the agar slants, and this was then added to 400 ml of sterile Hughes' media. Two milliliters of the inoculated media was then added to each of several 12 ml disposable vials.

The test compounds of formula IXA were formulated for evaluation by dissolving 10 mg of compound in a solution of 0.5 ml of acetone and 4.5 ml of sterile 0.1% aqueous polyoxyethylene sorbitan monooleate (Tween 80). Aliquot portions (10 ml) of the formulated test compounds were then added to the vials containing the algae species, Chlorella, Scenedesmus, Anacystis, and Anabaena flos-aquae, for a 10 ppm screen. Compounds that were considered active at 10 ppm were tested using all of the algae species at concentrations of 1 ppm and 0.5 ppm. These compounds were formulated by adding 1.0 ml of acetone and 11.0 ml of sterile 0.1% aqueous polyoxyethylene sorbitan monooleate (Tween 80). Aliquots of 0.1 ml and 0.05 ml each were added to 10 ml of inoculated media to obtain 1 ppm and 0.5 ppm, respectively. Visual observations and comparisons to non-treated control vials were made 7 days after treatment. Activity ratings were made on a scale of 1 to 5 according to the following meanings:

1 = no effect
2 = slight effect
3 = moderate effect
4 = heavy effect
5 = 100% control.

Table I which follows presents the algicidal activity of compounds of formula IXA evaluated according to the foregoing procedures.

## Table I

## Aquatic Algicide Activity

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 1 | 10 | 5 | 5 | 5 | | | 5 | | |
| | 1 | 4 | 5 | 5 | 3 | 5 | 5 | 5 | 5 |
| | 0.5 | 3 | 5 | 5 | 2 | 5 | 5 | 5 | 4 |
| 2 | 10 | 5 | 5 | 5 | | | 5 | | |
| | 1 | 4 | 5 | 5 | 4 | 5 | 5 | 4 | 5 |
| | 0.5 | 1 | 5 | 4 | 3 | 5 | 5 | 4 | 4 |
| 3 | 10 | 4 | 5 | 5 | | | 5 | | |
| | 1 | 3 | 5 | 4 | 4 | 5 | 5 | 4 | 4 |
| | 0.5 | 3 | 5 | 4 | 3 | 4 | 4 | 4 | 3 |
| 4 | 0.5 | 4 | 5 | 5 | 5 | 5 | 5 | 5 | 4 |
| 5 | 10 | 4 | 5 | 5 | | | 1 | | |
| | 1 | 4 | 5 | 5 | 5 | 4 | 5 | 4 | 2 |
| | 0.5 | 2 | 5 | 4 | 4 | 4 | 4 | 4 | 1 |
| 6 | 10 | 5 | 5 | 5 | | | 5 | | |
| | 1 | 5 | 5 | 5 | 5 | 5 | 5 | 4 | 4 |
| | 0.5 | 4 | 5 | 5 | 5 | 5 | 5 | 4 | 1 |

Table I continued

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 7 | 10 | 5 | 5 | 5 | | | 5 | | |
| | 10 | 5 | 5 | 5 | | | 5 | | |
| | 10 | 5 | 5 | 5 | | | 5 | | |
| | 1 | 4 | 5 | 5 | 4 | 5 | 4 | 4 | 1 |
| | 0.5 | 3 | 5 | 4 | 4 | 5 | 3 | 4 | 1 |
| 8 | 0.5 | 2 | 1 | 3 | 5 | 4 | 3 | 5 | 2 |
| 9 | 10 | 5 | 5 | 5 | | | 5 | | |
| | 1 | 4 | 5 | 5 | 5 | 4 | 5 | 5 | 4 |
| | 0.5 | 3 | 5 | 5 | 4 | 2 | 3 | 5 | 2 |
| 11 | 10 | 5 | 5 | 5 | | | 5 | | |
| | 1 | 5 | 5 | 5 | 5 | 5 | 5 | 5 | 5 |
| | 0.5 | 5 | 5 | 5 | 5 | 4 | 5 | 5 | 5 |
| 12 | 10 | 4 | 4 | 4 | | | 5 | | |
| | 1 | 3 | 5 | 5 | 1 | 1 | 5 | 1 | 3 |
| | 0.5 | 2 | 5 | 5 | 1 | 1 | 5 | 1 | 3 |
| 13 | 10 | 4 | 4 | 4 | | | 5 | | |
| | 1 | 3 | 4 | 4 | 4 | 4 | 4 | 4 | 1 |
| | 0.5 | 1 | 4 | 4 | 4 | 4 | 4 | 4 | 1 |

Table I continued

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 14 | 10 | 3 | 5 | 5 | | | 5 | | |
| | 1 | | 5 | 3 | 2 | 4 | 3 | 5 | 4 |
| | 0.5 | | 1 | 1 | 1 | 1 | 1 | 5 | 3 |
| 15 | 10 | 3 | 4 | 4 | | | 4 | | |
| | 1 | 1 | 5 | 2 | 5 | 2 | | 5 | 4 |
| | 0.5 | 1 | 1 | 2 | 1 | | 2 | 5 | 1 |
| 16 | 10 | 3 | 5 | 5 | | | 5 | | |
| | 1 | 1 | 4 | 4 | 4 | | 4 | 4 | 1 |
| | 0.5 | 1 | 4 | 4 | 4 | | 4 | 4 | 1 |
| 17 | 10 | 5 | 5 | 5 | | 5 | | | |
| | 1 | 5 | 5 | 5 | 5 | 5 | 5 | 5 | 5 |
| | 0.5 | 4 | 5 | 5 | 5 | 5 | 5 | 5 | 5 |
| 18 | 10 | 5 | 5 | 5 | | | 5 | | |
| | 1 | 1 | 5 | 4 | 4 | | 5 | 4 | 4 |
| | 0.5 | 3 | 5 | 1 | 1 | | 1 | 4 | 3 |
| 19 | 10 | 4 | 4 | 5 | | | 5 | | |
| | 10 | 5 | 5 | 5 | | | 5 | | |
| | 1 | 4 | 5 | 5 | 5 | | 5 | 4 | 4 |
| | 1 | 5 | 5 | 5 | 5 | 4 | 5 | 4 | 4 |
| | 0.5 | 4 | 5 | 4 | 5 | 4 | 4 | 4 | 3 |
| | 0.5 | 4 | 5 | 4 | 4 | | 5 | 4 | 4 |

Table I continued

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 20 | 10 | 2 | 5 | 5 | | | 4 | | |
| | 1 | 4 | 5 | 5 | 1 | 5 | 4 | 4 | 4 |
| | 0.5 | 2 | 4 | 4 | 1 | 3 | 1 | 4 | 3 |
| 21 | 10 | 4 | 5 | 5 | | | 4 | | |
| | 1 | 4 | 5 | 5 | 2 | 5 | 4 | 4 | 1 |
| | 0.5 | 4 | 5 | 5 | 1 | 4 | 4 | 4 | 1 |
| 22 | 10 | 5 | 5 | 5 | | 5 | | | |
| | 1 | 4 | 5 | 5 | 4 | 4 | 5 | 4 | 1 |
| | 0.5 | 3 | 5 | 4 | 4 | 3 | 5 | 4 | 1 |
| 23 | 10 | 5 | 5 | 5 | | | 5 | | |
| | 1 | 4 | 5 | 4 | 5 | 5 | 5 | 5 | 4 |
| | 0.5 | 4 | 4 | 4 | 4 | 5 | 1 | 4 | 3 |
| 24 | 10 | 5 | 4 | 4 | | | 4 | | |
| | 1 | 5 | 5 | 5 | 4 | 5 | 5 | 4 | 4 |
| | 0.5 | 4 | 5 | 5 | 4 | 4 | 5 | 4 | 4 |
| 25 | 10 | 5 | 1 | 5 | | | 5 | | |
| | 1 | 4 | 5 | 5 | 4 | 4 | 3 | 4 | 4 |
| | 0.5 | 2 | 5 | 3 | 2 | 3 | 2 | 4 | 4 |

Table I continued

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 26 | 10 | 5 | 5 | 5 | | | 5 | | |
| | 1 | 5 | 5 | 5 | 5 | 5 | | 5 | 4 |
| | 0.5 | 5 | 5 | 5 | 5 | 4 | 5 | 5 | 3 |
| 27 | 10 | 1 | 5 | 5 | | 3 | | | |
| 28 | 10 | 5 | 4 | 4 | | | 5 | | |
| | 1 | 1 | 5 | 5 | 1 | 4 | 4 | 5 | 5 |
| | 0.5 | 1 | 5 | 4 | 1 | 3 | 1 | 5 | 4 |
| 29 | 10 | 2 | 4 | 4 | | | 4 | | |
| | 1 | | | | 4 | 3 | | 4 | 4 |
| | 1 | 3 | 4 | 4 | | | 4 | | |
| | 0.5 | | | | 4 | 3 | | 4 | 4 |
| | 0.5 | 3 | 4 | 4 | | | 4 | | |
| 30 | 10 | 4 | 4 | 4 | | | 4 | | |
| | 1 | | | | 4 | 5 | | 4 | 4 |
| | 1 | 4 | 5 | 5 | | | 5 | | |
| | 0.5 | | | | 4 | 4 | | 4 | 4 |
| | 0.5 | 4 | 4 | 4 | | | 4 | | |
| 31 | 10 | 1 | 4 | 4 | | | 4 | | |
| | 1 | | | | 4 | 3 | | 4 | 4 |
| | 1 | 2 | 4 | 4 | | | 4 | | |
| | 0.5 | | | | 2 | 3 | | 4 | 4 |
| | 0.5 | 2 | 3 | 3 | | | 4 | | |

Table I continued

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 33 | 10 | 4 | 4 | 4 | | | 5 | | |
| | 1 | 4 | 4 | 4 | | | 4 | | |
| | 1 | | | | 4 | 4 | | 4 | 4 |
| | 0.5 | | | | 4 | 4 | | 4 | 4 |
| | 0.5 | 4 | 4 | 4 | | | 4 | | |
| 34 | 10 | 1 | 2 | 1 | | | 1 | | |
| 35 | 10 | 5 | 5 | 5 | 5 | 5 | | 5 | 1 |
| | 10 | | | | 5 | 5 | | 5 | 2 |
| | 1 | 4 | 5 | 5 | | | | | |
| | 1 | 3 | 5 | 5 | 5 | 5 | 5 | 5 | 3 |
| | 1 | 5 | 5 | 5 | | | 5 | | |
| | 0.5 | 4 | 5 | 5 | | | | | |
| | 0.5 | 3 | 5 | 5 | 5 | 5 | 5 | 1 | 2 |
| | 0.5 | 4 | 5 | 5 | | | 1 | | |
| 36 | 10 | 5 | 5 | 5 | | | 4 | | |
| | 10 | 3 | 4 | 5 | | | 4 | | |
| | 1 | 1 | 4 | 1 | 1 | 3 | 1 | 5 | 4 |
| | 0.5 | 1 | 4 | 1 | 1 | 2 | 1 | 5 | 3 |

Table I continued

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 37 | 10 | 5 | 5 | 5 | | | 5 | | |
| | 10 | 5 | 5 | 5 | | | 5 | | |
| | 1 | 1 | 5 | 3 | 1 | 3 | 3 | 5 | 3 |
| | 1 | 1 | 5 | 3 | 1 | 2 | 1 | 4 | 4 |
| | 0.5 | 1 | 4 | 3 | 1 | 1 | 1 | 5 | 2 |
| | 0.5 | 1 | 4 | 2 | 1 | 2 | 1 | 5 | 1 |
| 38 | 10 | 4 | 4 | 4 | | | 4 | | |
| | 1 | 1 | 1 | 1 | 1 | 3 | 3 | 3 | 2 |
| | 0.5 | 1 | 1 | 1 | 2 | 4 | 1 | 2 | 1 |
| 39 | 10 | 5 | 5 | 5 | | | 5 | | |
| | 1 | 1 | 1 | 4 | | | 1 | | |
| | 0.5 | 1 | 1 | 3 | | | 1 | | |
| 40 | 10 | 3 | 5 | 5 | | | 1 | | |
| | 1 | 1 | 2 | 2 | 1 | 1 | 1 | 1 | 1 |
| | 0.5 | 1 | 1 | 2 | 1 | 1 | 1 | 1 | 1 |
| 41 | 10 | 1 | 5 | 4 | | | 4 | | |
| | 1 | 1 | 2 | 1 | 1 | 1 | 1 | 1 | 1 |
| | 1 | | 1 | 1 | 1 | 1 | 2 | 1 | 1 |
| | 0.5 | 1 | 1 | 1 | 1 | 1 | 1 | 1 | 1 |
| | 0.5 | | 1 | 1 | 1 | 1 | 2 | 1 | 1 |

Table I continued

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 42 | 10 | 1 | 4 | 2 | | | 1 | | |
| 43 | 10 | 3 | 4 | 4 | | | 4 | | |
| | 1 | | | | 2 | 2 | | 4 | 3 |
| | 1 | 2 | 4 | 3 | | | 3 | | |
| | 0.5 | | | | 2 | 2 | | 4 | 3 |
| | 0.5 | 2 | 3 | 3 | | | 3 | | |
| 44 | 10 | 5 | 5 | 5 | | | 5 | | |
| | 1 | | 5 | 5 | 5 | 5 | 5 | 5 | 5 |
| | 0.5 | 3 | 5 | 5 | 3 | 4 | 5 | 5 | 4 |
| 45 | 10 | 4 | 5 | 5 | | | 5 | | |
| | 1 | 5 | 5 | 5 | 5 | 4 | 5 | 4 | 4 |
| | 0.5 | 4 | 4 | 4 | 4 | 4 | 5 | 4 | 4 |
| 46 | 10 | 5 | 5 | 5 | | | 5 | | |
| | 1 | 2 | 5 | 5 | 4 | 4 | 4 | 5 | 4 |
| | 0.5 | 1 | 4 | 4 | 2 | 3 | 2 | 5 | 1 |

Several of the compounds of formula IXA were evaluated in a secondary aquatic algicide screen. The test was carried out as described above, except that lower levels of compound were employed. The results of the secondary screen are reported in Table II which follows.

## Table II

Aquatic Algicide Activity

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 1 | .05 | 1 | 3 | 1 | 1 | 1 | 1 | 4 | 1 |
| | .1 | 1 | 4 | 1 | 1 | 1 | 1 | 4 | 4 |
| | .5 | 1 | 5 | 5 | 1 | 5 | 3 | 5 | 4 |
| 2 | .05 | 1 | 1 | 1 | 1 | 1 | 1 | 1 | 1 |
| | .1 | 1 | 3 | 1 | 1 | 1 | 1 | 2 | 1 |
| | .5 | 1 | 5 | 4 | 1 | 3 | 4 | 4 | 3 |
| 3 | .05 | 1 | 1 | 1 | 1 | 1 | 1 | 2 | 1 |
| | .1 | 1 | 1 | 1 | 1 | 1 | 1 | 3 | 1 |
| | .5 | 1 | 4 | 4 | 3 | 4 | 4 | 5 | 3 |
| 4 | .05 | 1 | 4 | 1 | 1 | 1 | 1 | 4 | 1 |
| | .1 | 1 | 5 | 4 | 1 | 2 | 3 | 5 | 1 |
| | .5 | 4 | 5 | 5 | 5 | 5 | 5 | 5 | 4 |
| 5 | .05 | 1 | 2 | 1 | 1 | 1 | 3 | 3 | 1 |
| | .1 | 1 | 3 | 1 | 3 | 1 | 3 | 3 | 1 |
| | .5 | 2 | 4 | 3 | 5 | 5 | 4 | 5 | 2 |
| 6 | .05 | 1 | 4 | 1 | 4 | 1 | 3 | 5 | 1 |
| | .1 | 1 | 4 | 2 | 4 | 4 | 3 | 5 | 1 |
| | .5 | 3 | 4 | 5 | 5 | 5 | 5 | 5 | 2 |

Table II continued

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 8 | .05 | 1 | 1 | 1 | 1 | 1 | 3 | 2 | 1 |
| | .1 | 1 | 1 | 1 | 3 | 1 | 3 | 2 | 2 |
| | .5 | 2 | 4 | 3 | 5 | 4 | 3 | 5 | 2 |
| 9 | .05 | 1 | 1 | 1 | 1 | 1 | 1 | 4 | 1 |
| | .1 | 1 | 1 | 1 | 1 | 1 | 1 | 5 | 1 |
| | .5 | 2 | 4 | 4 | 4 | 2 | 1 | 5 | 1 |
| 11 | .05 | 1 | 5 | 1 | 1 | 4 | 1 | 5 | 1 |
| | .1 | 3 | 5 | 1 | 1 | 4 | 1 | 5 | 1 |
| | .5 | 5 | 5 | 5 | 5 | 5 | 5 | 5 | 4 |
| 12 | .05 | 3 | 4 | 4 | 4 | 1 | 4 | 3 | 2 |
| | .1 | 2 | 4 | 4 | 5 | 3 | 4 | 4 | 2 |
| | .5 | 4 | 4 | 5 | 5 | 3 | 4 | 4 | 4 |
| 13 | .05 | 1 | 4 | 4 | 3 | 1 | 2 | 2 | 3 |
| | .1 | 1 | 4 | 4 | 4 | 2 | 2 | 3 | 4 |
| | .5 | 1 | 4 | 5 | 4 | 4 | 4 | 3 | 1 |
| 15 | .05 | 2 | 3 | 2 | 1 | 1 | 1 | 1 | 1 |
| | .1 | 1 | 3 | 2 | 3 | 2 | 2 | 1 | 1 |
| | .5 | 3 | 4 | 4 | 4 | 3 | 2 | 3 | 3 |

## Table II continued

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 16 | .05 | 1 | 2 | 2 | 3 | 1 | 1 | 1 | 1 |
| | .1 | 2 | 4 | 4 | 3 | 2 | 2 | 2 | 1 |
| | .5 | 1 | 4 | 4 | 4 | 3 | 2 | 3 | 1 |
| 17 | .05 | 1 | 4 | 1 | 1 | 1 | 1 | 1 | 1 |
| | .1 | 1 | 4 | 1 | 1 | 1 | 1 | 2 | 1 |
| | .5 | 4 | 5 | 5 | 4 | 5 | 5 | 4 | 3 |
| 19 | .05 | 1 | 3 | 1 | 2 | 1 | 4 | 3 | 1 |
| | .1 | 1 | 4 | 1 | 4 | 1 | 3 | 4 | 1 |
| | .5 | 3 | 4 | 4 | 5 | 5 | 5 | 5 | 3 |
| 21 | .05 | 1 | 3 | 1 | 2 | 1 | 3 | 3 | 1 |
| | .1 | 1 | 4 | 1 | 3 | 1 | 3 | 3 | 1 |
| | .5 | 2 | 4 | 5 | 3 | 5 | 3 | 5 | 1 |
| 22 | .05 | 1 | 3 | 1 | 2 | 1 | 3 | 2 | 1 |
| | .1 | 1 | 4 | 1 | 3 | 1 | 3 | 2 | 2 |
| | .5 | 2 | 4 | 3 | 4 | 5 | 3 | 5 | 2 |
| 23 | .05 | 1 | 1 | 1 | 1 | 1 | 1 | 2 | 2 |
| | .1 | 1 | 3 | 1 | 1 | 2 | 1 | 3 | 2 |
| | .5 | 3 | 4 | 4 | 4 | 5 | 4 | 4 | 3 |

Table II continued

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 24 | .05 | 1 | 3 | 1 | 3 | 1 | 3 | 4 | 1 |
| | .1 | 1 | 4 | 1 | 3 | 1 | 4 | 5 | 4 |
| | .5 | 3 | 4 | 5 | 4 | 5 | 5 | 5 | 4 |
| 26 | .05 | 1 | 4 | 1 | 1 | 1 | 2 | 5 | 1 |
| | .1 | 3 | 5 | 3 | 1 | 1 | 3 | 5 | 2 |
| | .5 | 4 | 5 | 5 | 5 | 3 | 5 | 5 | 3 |
| 28 | .05 | 2 | 4 | 3 | 4 | 1 | 3 | 3 | 1 |
| | .1 | 2 | 4 | 4 | 4 | 3 | 2 | 3 | 2 |
| | .5 | 4 | 4 | 5 | 4 | 4 | 4 | 3 | 1 |
| 35 | .05 | 1 | 4 | 4 | 2 | 4 | 4 | 5 | 1 |
| | .1 | 2 | 4 | 5 | 4 | 4 | 4 | 5 | 2 |
| | .5 | 4 | 5 | 5 | 5 | 5 | 5 | 5 | 3 |
| 44 | .05 | 1 | 4 | 4 | 4 | 1 | 2 | 2 | 1 |
| | .1 | 1 | 4 | 4 | 4 | 3 | 2 | 3 | 4 |
| | .5 | 3 | 4 | 4 | 4 | 4 | 4 | 3 | 1 |
| 45 | .05 | 1 | 1 | 1 | 1 | 1 | 1 | 5 | 2 |
| | .1 | 1 | 1 | 1 | 1 | 1 | 1 | 5 | 2 |
| | .5 | 1 | 5 | 3 | 1 | 4 | 5 | 5 | 3 |

The compounds of formula IXA have also been evaluated as an algicide on planktonic algae under field conditions in 16 liter buckets maintained outdoors and containing a layer of earth at the bottem. The buckets were filled with well-water, and then innoculated with green algae from slants (Scenedsmus). The algae bloom occurred in an untreated control in 24 hours.

The compounds of formula IXA were formulated as a technical solution of 1:9 ratio by volume of acetone:0.1% by volume of Tween 80. The appropriate volume of water was added to the formulation to obtain the solutions containing 0.1 to 1.0 ppm of the active compound of formula IXA. The formulation was added to the bucket 24 hours after innoculation. The activity was determined by visual observation based upon non-treated controls. Plant injury ratings were made visually in a scale of 0-10 with 0 being no injury and 10 being plant death. The injury rating was multiplied by 10 to obtain a percent inhibition. The results of these tests are reported in Table III.

Table III

ALGAE CONTROL-FIELD DATA

PERCENT INHIBITION-DAYS AFTER TREATMENT

| Compound of Example No. | Concentration ppm | DAY 4 | DAY 7 |
|---|---|---|---|
| 1 | 0.1 | 10.0 | 30.0 |
| | 0.3 | 16.7 | 46.7 |
| | 1.0 | 28.3 | 76.7 |
| 11 | 0.1 | 33.3 | 60.0 |
| | 0.3 | 31.7 | 73.3 |
| | 1.0 | 36.7 | 93.3 |
| 17 | 0.1 | 20.0 | 60.0 |
| | 0.3 | 40.0 | 86.7 |
| | 1.0 | 30.0 | 80.0 |
| 45 | 0.1 | 23.0 | 0.0 |
| | 0.3 | 26.7 | 53.3 |
| | 1.0 | 30.0 | 83.3 |
| Control | 0 | 10.0 | 0.0 |

Terrestrial Herbicidal Method

A method of controlling the growth of unwanted terrestrial vegetation comprises contacting the vegetation to be controlled or the soil in which the vegetation is growing with a herbicidally-effective amount of a compound of the formula

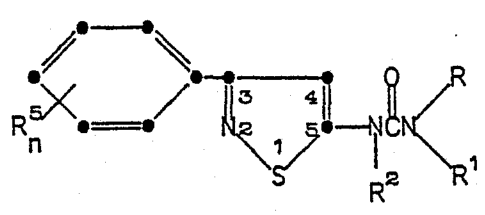

X

wherein

R and $R^1$ are $C_1$-$C_6$ alkyl, $C_3$-$C_6$ alkenyl, or $C_1$-$C_2$ alkoxy, provided that one of R or $R^1$ is methyl;

$R^2$ is hydrogen or $C_1$-$C_3$ alkyl;

n is 1 or 2; and

$R^5$ is hydrogen, halo, methoxy, or $C_1$-$C_3$ alkyl; or an agriculturally acceptable acid-addition salt thereof; provided that when R and $R^1$ are methyl, and $R^2$ is hydrogen, $R^5$ cannot be 3-methyl. This means that the compound cannot be N'-[3-(3-methylphenyl)-5-isothiazolyl]-N,N-dimethylurea.

More preferred compounds of formula X within this method are those in which:

R and $R^1$ are $C_1$-$C_4$ alkyl or $C_1$-$C_2$ alkoxy; provided that one of R and $R^1$ is methyl;

$R^5$ is hydrogen or 4-fluoro; and

n is 1.

Even more preferred compounds of formula X are where:

R is $C_1$-$C_3$ alkyl; $R^1$ is methoxy or methyl; provided that one of R and $R^1$ is methyl; and

$R^5$ is 4-fluoro.

Most preferred compounds of formula X are:

N'-[3-(4-fluorophenyl)-5-isothiazolyl]-N'-ethyl-N-methyl-N-(1-methylethyl)urea;

N'-[3-(4-fluorophenyl)-5-isothiazolyl]-N',N,N-trimethylurea;

N'-[3-(4-fluorophenyl)-5-isothiazolyl]-N-ethyl-N-methylurea;

N'-[3-(4-fluorophenyl)-5-isothiazolyl]-N-methoxy-N-methylurea; and

N'-[3-(4-fluorophenyl)-5-isothiazolyl]-N-methoxy-N-methylurea monohydrochloride.

The isothiazolylureas provided by formula X exhibit both terrestrial and aquatic herbicidal activity and accordingly are useful in the control and elimination of unwanted vegetative growth.

The herbicides of formula X are effective terrestrially in both preemergent and postemergent control of a wide variety of grasses and broadleaf weeds. Commonly encountered unwanted terrestrial vegetation which is subject to control with the herbicidal ureas of formula X include:

Wild Oats (*Avena fatua*)
Catchweed Bedstraw (*Galium aparine*)
Scentless Mayweed (*Matricaria inodora*)
Ladysthumb (*Polygonum persicaria*)
Common Chickweed (*Stellaria media*)
Ivyleaf Speedwell (*Veronica hederaefolia*)
Blackgrass (*Alopecurus myosuroides*)
Chrysanthemum (*Chrysanthemum spp.*)
Common Purslane (*Portulaca oleracea*)
Sida (*Sida spp.*)
Bristly Starbur (*Acanthospermum hispidum*)
Goosegrass (*Eleusine indica*)
Smooth Pigweed (*Amaranthus hybridus*)
Alexandergrass (*Brachiaria plantaginea*)

Tall Morningglory (Ipomoea purpurea)
Common Lambsquarters (Chenopodium album)
Green Smartweed (Polygonum scabrum)
Green Foxtail (Setaria viridis)
Redroot Pigweed (Amaranthus retroflexus)
Wild Buckwheat (Polygonum convolvulus)
Brazil Calalilly (Richardia brasiliensis)
Natal Grass (Rhynchelytrum roseum)
Ryegrass (Lolium rigidum)
Kapeweed (Cryptostemma calendula)
Purple Loosestrife (Lythrum salicaria)
Wild Radish (Raphanus raphanistrum)
Wireweed (Polygonum aviculare)
Henbit (Lamium amplexicaule)
Wild Mustard (Brassica kaber)
Barnyard Grass (Echinochloa crus-galli)
Foxtail Millet (Setaria italica)
Velvetleaf (Abutilon theophrasti)
Indian Mustard (Brassica juncea)
Birdseye Speedwell (Veronica persica)
Canada Thistle (Cirsium arvense)
Wild Chamomile (Matricaria chamomilla)
Annual Bluegrass (Poa annua)
Buttercup (Ranunculus spp.)
Field Speedwell (Veronica agrestis)
Field Violet (Viola arvensis)
Field Pennycress (Thlaspi arvense)
Wild Violet (Viola tricolor)
Shirley Poppy (Papaver rhoeas)
Field Poppy (Papaver dubium)

Foolsparsley (Aethusa cynapium)
Field Chickweed (Cerastium arvense)
Southern Sandbur (Cenchrus echinatus)
Large Crabgrass (Digitaria sanguinalis)
Cheat (Bromus secalinus)
Morningglory (Ipomea spp.)
Common Ragweed (Ambrosia artemisiifolia)
Common Milkweed (Asclepias syriaca)
Giant Foxtail (Setaria faberi)
Common Cocklebur (Xanthium pensylvanicum)
Spurred Anoda Anoda cristata)
Sicklepod (Cassia obtusifolia)
Yellow Nutsedge Cyperus esculentus)
Jimsonweed (Datura stramonium)
Prickly Sida (Sida spinosa)
Corn Gromwell (Lithospermum arvense)
Yellow Foxtail (Setaria glauca)
Tansymustard (Descurainia pinnata)
Pepperweed (Lepidium spp.)
Bromegrass Bromus spp.)
Garden Spurge (Euphorbia hirta)
Crowfootgrass (Dactyloctenium aegyptium)
Florida Beggarweed (Desmodium tortuosum)
Spotted Spurge (Euphorbia maculata)
Smallflower Morningglory (Jacquemontia tamnifolia)
Browntop Millet (Panicum ramosum)
Coast Fiddleneck (Amsinckia intermedia)
Wild Turnip (Brassica campestris)
Black Mustard (Brassica nigra)
Shepherdspurse (Capsella bursa-pastoris)

Italian Ryegrass (*Lolium multiflorum*)
London Rocket (*Sisymbrium irio*)
Redmaids Rockpurslane (*Calandrinia caulescens*)
Common Groundsel (*Senecio vulgaris*)
Ivyleaf Morningglory (*Ipomoea hederacea*)
Fall Panicum (*Panicum dichotomiflorum*)
Powell Amaranth (*Amaranthus powellii*)
Texas Panicum (*Panicum texanum*)
Hemp Sesbania (*Sesbania exaltata*)
Annual Sowthistle (*Sonchus oleraceus*)
Field Bindweed (*Convolvulus arvensis*)
Erect Knotweed (*Polygonum erectum*)
Venice Mallow (*Hibiscus trionum*)
Zinnia (*Zinnia elegens*)
Nightshade (*Solanum spp.*)

The present compounds of formula X have also been found safe on a wide variety of desirable plant species, thereby exhibiting their unique selective capacity. Representative examples of relatively tolerant plant species, depending on the concentration of active compounds of formula X employed, include the following:

Corn (*Zea mays*)
Wheat (*Triticum aestivum*)
Soybean (*Glycine max*)
Rice (*Oryza sativa*)
Barley (*Hordeum vulgare*)
Cotton (*Gossypium hirsutum*)
Sorghum (*Sorghum vulgare v. saccharatum*)

Sugarcane (*Saccharum* *officinarum*)
Peanut (*Arachis* *hypogaea*)
Alfalfa (*Medicago* *sativa*)
Cucumber (*Cucumis* *sativus*)
Tomato (*Lycopersicon* *esculentum*)
Sugar beets (*Beta* *vulgaris*)

A test used to evaluate herbicidal efficacy was conducted at a compound concentration of 15 pounds per acre (16.8 kilograms per hectare). In this test a standard sand:soil mixture (1:1) was sterilized and added to separate containers and tomato, large crabgrass, and pigweed seeds were planted by row.

The test compounds of formula X were formulated for application by dissolving the compound into a solvent, containing acetone, ethanol, and a blend of anionic and nonionic surfactants. The solvent/compound solution was diluted with deionized water and applied post-emergence to some planted containers and preemergence to others. Postemergent treatment was made 11 to 13 days after planting, while preemergent treatment was made one day after planting.

Following treatment the containers were moved to the greenhouse and watered as necessary. Observations were made 10 to 13 days after treatment using untreated control plants as standards. The degree of herbicidal activity was determined by rating the treated plants on a scale of 1 to 5. On this scale "1" indicates no injury, "2" is slight injury, "3" is moderate injury, "4" is severe injury, and "5" indicates death

to the plant or no seedling emergence. Also, the various types of injury of each test species were coded as follows.

A = abscission of leaves
B = burned
C = chlorosis
D = death
E = epinasty
F = formation effects
G = dark green
I = increased plant growth
L = local necrosis
N = no germination
P = purple pigmentation
R = reduced germination
S = stunting
U = unclassified injury

Table IV which follows presents the terrestrial herbicidal activity of the compound of formula X at 15 pounds per acre (16.8 kilograms per hectare). Table V which follows shows activity at 8 pounds per acre (9 kilograms per hectare).

Table IV

Terestrial Herbicidal Activity

| Compound of Example No. | Plant Species | | | | | |
|---|---|---|---|---|---|---|
| | Pre-emergence | | | Post-emergence | | |
| | Tomato | Large Crab-grass | Pig-weed | Tomato | Large Crab-grass | Pig-weed |
| 1 | 4BS | 4RS | 4RS | 5D | 5D | 5D |
| 2 | 1 | 2S | 2S | 5D | 4BS | 5D |
| 3 | 1 | 1 | 2RS | 4BS | 4BS | 4BS |
| 9 | 1 | 1 | 1 | 5D | 5D | 5D |
| 10 | 2CS | 4RS | 4RS | 5D | 4BS | 5D |
| 11 | 2CS | 1 | 1 | 4FS | 5D | 5D |
| 15 | 1 | 1 | 1 | 4BS | 4BS | 4BS |
| 17 | 1 | 1 | 1 | 5D | 3BS | 5D |
| 18 | 1 | 1 | 3RS | 5D | 4BS | 5D |
| 19 | 1 | 1 | 1 | 3BS | 2BS | 3BS |
| 21 | 1 | 1 | 1 | 5D | 3BS | 5D |
| 22 | 2RS | 1 | 2RS | 5D | 4BS | 5D |
| 24 | 1 | 1 | 3RS | 5D | 4BS | 5D |
| 25 | 1 | 1 | 1 | 5D | 5D | 5D |
| 26 | 1 | 1 | 2S | 5D | 4BS | 5D |
| 28 | 1 | 2RS | 1 | 5D | 5D | 5D |
| 35 | 1 | 1 | 1 | 5D | 5D | 5D |
| 36 | 2CS | 1 | 2RS | 4CBS | 5D | 4CS |
| 37 | 2CS | 2CS | 2S | 5D | 4BS | 5D |
| 38 | 1 | 2S | 1 | 4BS | 4BS | 4BS |
| 41 | 1 | 1 | 1 | 1 | 1 | 1 |
| 42 | 1 | 1 | 1 | 1 | 1 | 1 |
| 44 | 5N | 4RS | 4RS | 5D | 4BS | 5D |
| 45 | 1 | 2RS | 3RS | 5D | 5D | 5D |

Table V

PLANT SPECIES

Post-emergence

| Compound of Example No. | Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|
| 2 | | | 1 | 1 | | 3 | 1 | | 2 | 2 | 3 |
| 3 | | | 1 | 1 | | 1 | 1 | | 1 | 1 | 1 |
| 5 | | | 1 | 2 | | 2 | 1 | | 3 | 2 | 3 |
| 6 | | | 1 | 3 | | 4 | 2 | | 4 | 3 | 4 |
| 22 | | | 1 | 2 | | 4 | 2 | | 4 | 3 | 4 |
| 24 | | | 1 | 4 | | 4 | 2 | | 4 | 3 | 4 |
| 25 | | | 1 | 4 | | 4 | 3 | | 4 | 4 | 4 |
| 45 | 4 | 3 | | 2 | 3 | 4 | 2 | 1 | 3 | 3 | 5 |

The herbicidal activity of some of the compounds of formula X was further evaluated at various application rates in a multiple species greenhouse test. Several additional weed and crop species were utilized to determine the herbicidal activity and selectivity of the test compounds. Lower concentrations of the test compounds were obtained by serial dilution of the above described formulation with a mixture of the surfactant and deionized water. The compounds were evaluated according to the general procedure outlined above. See Tables VI and VII.

## Table VI

PLANT SPECIES

| Compound of Example No. | appln. rate lbs/A | Pre-emergence | | | | | | | | | | | | | | | | | | | | Post-emergence | | | | | | | | | | |
|---|---|---|---|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Tomato | Barnyard Grass | Corn | Large Crabgrass | Mustard | Pigweed | Foxtail | Wild Oats | Velvetleaf | Morningglory | Zinnia |
| 1 | 1 | 1 | 1 | 1 | 1 | 3 | 2 | 1 | 3 | 3 | 2 | 4 | 3 | 2 | 4 | 1 | 2 | 3 | 3 | 3 | 1 | | | | | | | | | | | |
| | 2 | 1 | 1 | 1 | 2 | 2 | 3 | 2 | 3 | 3 | 2 | 4 | 4 | 4 | 4 | 4 | 1 | 4 | 3 | 4 | 1 | | | | | | | | | | | |
| | 4 | 1 | 1 | 2 | 1 | 4 | 4 | 2 | 3 | 4 | 2 | 4 | 4 | 4 | 5 | 5 | 2 | 4 | 3 | 5 | 3 | | | | | | | | | | | |
| 9 | 1 | | | | | | | | | | | | | | | | | | | | | | | 1 | 2 | | 3 | 1 | | 2 | 2 | 2 |
| | 2 | | | | | | | | | | | | | | | | | | | | | | | 1 | 3 | | 4 | 2 | | 3 | 3 | 3 |
| | 4 | | | | | | | | | | | | | | | | | | | | | | | 1 | 3 | | 4 | 2 | | 3 | 3 | 4 |
| 10 | 1 | 1 | 1 | 1 | | 1 | 1 | 1 | 1 | 1 | 1 | | 4 | 3 | | 4 | 2 | 1 | 1 | 1 | 2 | | | 1 | 2 | | 2 | 3 | | 4 | 3 | 3 |
| | 2 | 1 | 1 | 1 | 1 | | 3 | 2 | 2 | 2 | 2 | | 4 | 2 | | 4 | 2 | 1 | 1 | 2 | 2 | | | 1 | 3 | | 2 | 3 | | 3 | 3 | 4 |
| | 4 | 1 | 1 | 1 | 1 | 1 | 3 | 2 | 2 | 1 | 2 | | 2 | 2 | | 1 | 3 | 1 | 1 | 1 | 1 | | | 1 | 2 | | 3 | 2 | | 4 | 4 | 4 |
| | 8 | 2 | | | | | | | | | | | 4 | | 5 | 4 | | 2 | | 3 | 1 | | | 1 | 4 | | 5 | 3 | | 4 | 3 | 4 |
| 11 | 1 | 1 | 1 | 1 | 1 | 1 | 1 | 1 | 1 | 1 | 1 | 1 | 1 | 1 | 1 | 1 | 1 | 1 | 1 | 1 | 1 | | | 1 | 4 | | 4 | 3 | | 3 | 2 | 4 |
| | 2 | 1 | 1 | 1 | 1 | 1 | 1 | 1 | 1 | 1 | 1 | 2 | 2 | 1 | 3 | 1 | 1 | 1 | 1 | 1 | 1 | | | 1 | 4 | | 4 | 3 | | 5 | 2 | 4 |
| | 4 | 1 | 1 | 1 | 1 | 1 | 3 | 1 | 1 | 2 | 1 | 2 | 2 | 2 | 3 | 1 | 1 | 1 | 1 | 1 | 1 | | | 1 | 4 | | 4 | 3 | | 4 | 2 | 4 |
| | 8 | 1 | | | | | | | | | | | 4 | | 5 | 1 | | 2 | | 1 | 1 | | | 1 | 2 | | 5 | 1 | | 4 | 2 | 3 |

Table VI cont'd.

PLANT SPECIES

| Compound of Example No. | appln. rate lbs/A | Pre-emergence Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 12 | 1 | 1 | 1 | 1 | 1 | 1 | 2 | 2 | 2 | 1 | 1 | 4 | 1 | 2 | 3 | 1 | 1 | 1 | 1 | 1 | 1 | 2 | 1 | | 3 | 3 | 5 | 2 | 1 | 2 | 2 | 4 |
| | 2 | 1 | 1 | 1 | 1 | 1 | 1 | 2 | 1 | 1 | 1 | 4 | 2 | 2 | 2 | 1 | 1 | 3 | 2 | 2 | 2 | 4 | 1 | | 3 | 3 | 4 | 3 | 1 | 2 | 2 | 3 |
| | 4 | 1 | 1 | 1 | 1 | 1 | 1 | 2 | 2 | 1 | 1 | 2 | 2 | 2 | 5 | 1 | 1 | 1 | 1 | 2 | 2 | 3 | 2 | | 3 | 3 | 5 | 4 | 1 | 3 | 2 | 4 |
| | 8 | | | | | | | | | 4 | 1 | | 3 | 2 | 2 | 2 | 1 | 4 | | 2 | 2 | 4 | 1 | | 3 | 4 | 4 | 3 | 1 | 3 | 3 | 3 |
| 13 | 1 | 1 | 1 | 1 | 1 | 1 | 1 | 1 | 1 | 1 | 1 | 4 | 3 | 1 | 1 | 2 | 1 | 1 | 1 | 1 | 1 | 4 | 1 | | 3 | 4 | 3 | 2 | 2 | 5 | 4 | 4 |
| | 2 | 1 | 1 | 2 | 2 | 1 | 3 | 1 | 2 | 1 | 1 | 4 | 4 | 3 | 3 | 3 | 1 | 3 | 4 | 1 | 3 | 4 | 3 | | 3 | 4 | 3 | 4 | 1 | 5 | 4 | 4 |
| | 4 | 1 | 1 | 2 | 4 | 3 | 4 | 2 | 3 | 2 | 3 | 4 | 4 | 2 | 2 | 3 | 2 | 4 | 3 | 3 | 3 | 3 | 1 | | 3 | 4 | 5 | 3 | 1 | 5 | 4 | 4 |
| | 8 | | | | | | | | | 4 | 2 | | 4 | 4 | 5 | 3 | 2 | 4 | | 4 | 4 | 4 | 2 | | 4 | 4 | 5 | 4 | 1 | 5 | 4 | 5 |
| 14 | 1 | 1 | 1 | 1 | 1 | 1 | 1 | 1 | 1 | 1 | 1 | 1 | 2 | 2 | 1 | 1 | 1 | 1 | 1 | 1 | 1 | 2 | 1 | | 2 | 4 | 3 | 1 | 1 | 2 | 2 | 2 |
| | 2 | 1 | 1 | 1 | 2 | 1 | 3 | 2 | 2 | 2 | 1 | 4 | 3 | 3 | 2 | 2 | 1 | 2 | 2 | 1 | 3 | 2 | 1 | | 2 | 4 | 4 | 2 | 1 | 2 | 3 | 2 |
| | 4 | 4 | 1 | 1 | 1 | 2 | 4 | 2 | 3 | 2 | 3 | 4 | 4 | 4 | 5 | 2 | 2 | 4 | 2 | 1 | 3 | 2 | 1 | | 2 | 4 | 4 | 2 | 1 | 3 | 3 | 3 |
| | 8 | | | | | | | | | 5 | 4 | | 5 | 4 | 5 | 4 | 3 | 4 | | 3 | 5 | 5 | 2 | | 4 | 5 | 5 | 3 | 1 | 4 | 4 | 5 |
| 15 | 8 | | | | | | | | | 2 | 1 | | 3 | 3 | 1 | 3 | 2 | 2 | | 2 | 2 | 3 | 1 | | 3 | 4 | 1 | 1 | 1 | 3 | 3 | 3 |
| 16 | 8 | | | | | | | | | 2 | 1 | | 3 | 3 | 1 | 3 | 2 | 2 | | 2 | 2 | 3 | 1 | | 3 | 4 | 1 | 1 | 1 | 3 | 3 | 3 |
| 17 | 8 | 1 | | | | | | | | | | | 1 | | 1 | 1 | | 1 | | 1 | 1 | | | 1 | 1 | | 3 | 2 | | 3 | 2 | 3 |
| 18 | 1 | | | | | | | | | | | | 4 | | 1 | 1 | | 3 | | 1 | 1 | | | | 1 | | 4 | 1 | | 1 | 2 | 2 |

## Table VI cont'd.

PLANT SPECIES

| Compound of Example No. | appln. rate lbs/A | Pre-emergence: Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence: Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 23 | 1 | 1 | 1 | 1 | 1 | 1 | 1 | 1 | 1 | 1 | 1 | | 1 | 1 | 1 | 1 | 1 | 1 | 1 | 1 | 1 | | | | | | | | | | | |
| | 2 | 1 | 1 | 1 | 1 | 1 | 1 | 1 | 1 | 1 | 1 | | 1 | 2 | 2 | 1 | 1 | 1 | 1 | 1 | 1 | | | | | | | | | | | |
| | 4 | 1 | 1 | 1 | 1 | 1 | 1 | 1 | 1 | 1 | 1 | 1 | 4 | 1 | 1 | 1 | 1 | 3 | 1 | 1 | 1 | | | | | | | | | | | |
| | 8 | 1 | | | | | | | | | | | 4 | | 4 | 3 | | 4 | | 1 | 1 | | | 1 | 1 | | 4 | 2 | | 2 | 2 | 1 |
| 24 | 1 | | | | | | | | | | | | | | | | | | | | | | | 1 | 1 | | 3 | 3 | | 1 | 2 | 2 |
| | 2 | | | | | | | | | | | | | | | | | | | | | | | 1 | 2 | | 4 | 2 | | 1 | 3 | 3 |
| | 4 | | | | | | | | | | | | | | | | | | | | | | | 1 | 1 | | 4 | 2 | | 1 | 3 | 3 |
| 25 | 1 | | | | | | | | | | | | | | | | | | | | | | | 1 | 4 | | 3 | 2 | | 4 | 4 | 4 |
| | 2 | | | | | | | | | | | | | | | | | | | | | | | 1 | 4 | | 4 | 4 | | 4 | 4 | 4 |
| | 4 | | | | | | | | | | | | | | | | | | | | | | | 2 | 4 | | 4 | 3 | | 4 | 4 | 4 |
| 27 | 8 | 1 | | | | | | | | | | | 1 | | 1 | 2 | | 2 | | 1 | 2 | | | 2 | 2 | | 2 | 2 | | 2 | 3 | 3 |
| 28 | .5 | 1 | 1 | 1 | 1 | 1 | 1 | 2 | 1 | 1 | 1 | 2 | 1 | 2 | 3 | 2 | 2 | 2 | 1 | 1 | 2 | 3 | 2 | | 4 | 2 | 4 | 2 | 2 | 4 | 5 | 5 |
| | 1 | 1 | 1 | 1 | 1 | 2 | 2 | 2 | 3 | 3 | 1 | 4 | 4 | 3 | 4 | 3 | 1 | 4 | 4 | 1 | 2 | 3 | 2 | | 3 | 4 | 5 | 4 | 2 | 5 | 5 | 4 |
| | 2 | 2 | 2 | 2 | 2 | 2 | 3 | 2 | 5 | 4 | 1 | 5 | 4 | 4 | 5 | 3 | 1 | 5 | 3 | 2 | 2 | 5 | 2 | | 3 | 3 | 5 | 4 | 2 | 5 | 5 | 4 |
| | 8 | | | | | | | | | 4 | 4 | | 4 | 4 | 5 | 4 | 1 | 4 | | 3 | 5 | 4 | 3 | | 4 | 5 | 4 | 4 | 1 | 4 | 5 | 4 |

Table VI cont'd.

PLANT SPECIES

Pre-emergence

| Compound of Example No. | appln. rate lbs/A | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia |
|---|---|---|---|---|---|---|---|---|---|---|---|---|---|---|---|---|---|---|---|---|---|
| 29 | 1 | 1 | 1 | 1 | 1 | 1 | 1 | 1 | 1 | 1 | 1 | 1 | 1 | 1 | 1 | 1 | 1 | 1 | 1 | 1 | 1 |
| | 2 | 1 | 1 | 1 | 1 | 1 | 2 | 2 | 1 | 1 | 1 | 1 | 1 | 1 | 2 | 2 | 1 | 1 | 2 | 1 | 1 |
| | 4 | 1 | 1 | 2 | 1 | 2 | 4 | 2 | 1 | 1 | 1 | 1 | 2 | 2 | 3 | 3 | 1 | 1 | 1 | 1 | 2 |
| | 8 | | | | | | | | | 3 | 1 | | | 5 | 5 | 4 | 2 | 4 | | 4 | 2 |
| 30 | 1 | 1 | 1 | 1 | 1 | 1 | 3 | 1 | 2 | 2 | 1 | 1 | 2 | 2 | 2 | 1 | 1 | 1 | 1 | 1 | 1 |
| | 2 | 1 | 1 | 4 | 1 | 1 | 2 | 2 | 2 | 1 | 1 | 4 | 2 | 3 | 1 | 1 | 1 | 1 | 1 | 1 | 1 |
| | 4 | 2 | 1 | 2 | 2 | 2 | 1 | 2 | 2 | 2 | 1 | 2 | 2 | 3 | 4 | 2 | 1 | 5 | 1 | 1 | 3 |
| | 8 | | | | | | | | | 4 | 1 | | | 4 | 5 | 4 | 1 | 2 | | 1 | 2 |
| 32 | 1 | 1 | 1 | 2 | 1 | 2 | 2 | 1 | 2 | 1 | 1 | 1 | | 2 | 1 | 1 | 1 | 1 | 2 | 1 | 1 |
| | 2 | 1 | 1 | 2 | 2 | 2 | 2 | 1 | 2 | 1 | 1 | 5 | | 4 | 5 | 4 | 1 | 2 | 5 | 1 | 1 |
| | 4 | 3 | 3 | 3 | 2 | 3 | 4 | 1 | 3 | 2 | 1 | 5 | | 5 | 5 | 4 | 2 | 4 | 5 | 2 | 2 |
| | 8 | | | | | | | | | 4 | 3 | | 5 | 5 | 5 | 4 | 1 | 4 | | 3 | 3 |

Post-emergence

| Compound of Example No. | appln. rate lbs/A | Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|
| 29 | 1 | | | | | | | | | | | |
| | 2 | | | | | | | | | | | |
| | 4 | | | | | | | | | | | |
| | 8 | | | | | | | | | | | |
| 30 | 1 | 2 | 1 | | 2 | 4 | 3 | 2 | 1 | 3 | 2 | 5 |
| | 2 | 3 | 1 | | 3 | 4 | 3 | 2 | 1 | 3 | 3 | 5 |
| | 4 | 4 | 2 | | 2 | 4 | 5 | 2 | 2 | 4 | 3 | 5 |
| | 8 | 4 | 2 | | | 4 | 3 | 3 | 2 | 3 | 3 | 4 |
| 32 | 1 | 1 | 2 | | 5 | 4 | 4 | 2 | 1 | 3 | 3 | 3 |
| | 2 | 2 | 2 | | 5 | 4 | 4 | 4 | 2 | 4 | 4 | 4 |
| | 4 | 4 | 2 | | 5 | 4 | 5 | 2 | 2 | 4 | 4 | 4 |
| | 8 | 5 | 4 | | 4 | 5 | 5 | 5 | 3 | 4 | 5 | 4 |

Table VI cont'd.

PLANT SPECIES

| Compound of Example No. | appln. rate lbs/A | Pre-emergence: Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence: Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 33 | 1 | 1 | 1 | 1 | 1 | 1 | 1 | 1 | 1 | 1 | 1 | 4 | 4 | 4 | 5 | 1 | 1 | 1 | 1 | 1 | 1 | 3 | 3 | | 2 | 4 | 3 | 3 | 2 | 2 | 4 | 4 |
| | 2 | | 1 | 1 | 1 | 5 | 3 | 1 | 3 | 5 | 1 | 4 | 3 | 1 | 5 | 2 | 1 | 1 | 3 | 1 | 1 | 3 | 2 | | 3 | 4 | 4 | 3 | 2 | 4 | 4 | 5 |
| | 4 | 2 | 1 | 2 | 1 | 3 | 3 | 1 | 3 | 2 | 1 | 5 | 4 | 4 | 5 | 5 | 2 | 2 | 2 | 4 | 1 | 4 | 3 | | 4 | 5 | 4 | 3 | 1 | 4 | 4 | 5 |
| | 8 | | | | | | | | | 3 | 3 | | 4 | 5 | 5 | 5 | 2 | 4 | | 5 | 5 | 2 | 1 | | 3 | 5 | 4 | 2 | 2 | 3 | 3 | 3 |
| 34 | 8 | | | | | | | | | 2 | 1 | | 1 | 1 | 2 | 1 | 1 | 1 | | 1 | 1 | 1 | 1 | | 1 | 1 | 1 | 1 | 1 | 1 | 1 | 1 |
| 35 | 8 | 2 | | | | | | | | | | | 1 | | 3 | 1 | | 1 | | 2 | 1 | | | 1 | 2 | | 4 | 1 | | 1 | 1 | 1 |
| 36 | 1 | 1 | 1 | 1 | 1 | 1 | 1 | 1 | 1 | 1 | 1 | 3 | 3 | 1 | 2 | 1 | 1 | 2 | 1 | 1 | 1 | | | | | | | | | | | |
| | 2 | 1 | 1 | 1 | 1 | 1 | 1 | 1 | 1 | 1 | 1 | 4 | 4 | 1 | 3 | 1 | 1 | 1 | 1 | 1 | 1 | | | | | | | | | | | |
| | 4 | 1 | 1 | 1 | 1 | 1 | 2 | 1 | 2 | 1 | 1 | 4 | 4 | 4 | 4 | 1 | 1 | 1 | 1 | 1 | 1 | | | | | | | | | | | |
| | 8 | 1 | | | | | | | | | | | 4 | | 5 | 3 | | 2 | | 1 | 1 | | | | 3 | | 3 | 2 | | 3 | 3 | 3 |
| 37 | 1 | 1 | 1 | 1 | 1 | 1 | 1 | 1 | 1 | 1 | 1 | 4 | 1 | 2 | 3 | 1 | 1 | 1 | 1 | 1 | 1 | | | 1 | 4 | | 4 | 4 | | 3 | 4 | 4 |
| | 2 | 1 | 1 | 1 | 1 | 1 | 3 | 1 | 1 | 2 | 1 | 4 | 3 | 3 | 5 | 3 | 1 | 1 | 1 | 1 | 1 | | | 1 | 4 | | 4 | 4 | | 3 | 4 | 4 |
| | 4 | 1 | 1 | 1 | 1 | 1 | 4 | 2 | 1 | 1 | 2 | 5 | 5 | 4 | 5 | 5 | 1 | 1 | 1 | 1 | 1 | | | 2 | 4 | | 4 | 4 | | 3 | 4 | 4 |
| | 8 | 1 | | | | | | | | | | | 5 | | 5 | 5 | | 1 | | 1 | 1 | | | 3 | 5 | | 5 | 4 | | 4 | 5 | 5 |

Table VI cont'd.

PLANT SPECIES

Pre-emergence

| Compound of Example No. | appln. rate lbs/A | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia |
|---|---|---|---|---|---|---|---|---|---|---|---|---|---|---|---|---|---|---|---|---|---|
| 38 | 1 | 1 | 1 | 1 | 1 | 1 | 1 | 1 | 1 | 1 | 1 | 5 | 1 | 3 | 2 | 1 | 1 | 2 | 3 | 1 | 1 |
| | 2 | 1 | 1 | 1 | 1 | 1 | 2 | 2 | 2 | 1 | 1 | 5 | 5 | 3 | 3 | 3 | 1 | 3 | 4 | 4 | 1 |
| | 4 | 1 | 5 | 1 | 3 | 1 | 4 | 2 | 3 | 1 | 1 | 5 | 4 | 2 | 2 | 2 | 1 | 4 | 4 | 3 | 3 |
| | 8 | | | | | | | | | 1 | 2 | | 5 | 3 | 5 | 1 | 1 | 2 | | 5 | 4 |
| 39 | 1 | | | | | | | | | | | | | | | | | | | | |
| | 2 | | | | | | | | | | | | | | | | | | | | |
| | 4 | | | | | | | | | | | | | | | | | | | | |
| | 8 | | | | | | | | | 2 | 2 | | 3 | 3 | 3 | 3 | 1 | 1 | | 1 | 2 |
| 40 | 1 | 1 | 1 | 1 | 1 | 1 | 1 | 1 | 1 | 1 | 1 | 5 | 4 | 3 | 4 | 3 | 1 | 3 | 2 | 2 | 1 |
| | 2 | 1 | 1 | 1 | 1 | 2 | 1 | 1 | 2 | 1 | 1 | 5 | 4 | 3 | 4 | 3 | 2 | 3 | 2 | 4 | 2 |
| | 4 | 1 | 1 | 1 | 1 | 1 | 1 | 1 | 1 | 1 | 1 | 4 | 5 | 4 | 5 | 2 | 1 | 1 | | 3 | 1 |
| | 8 | | | | | | | | | 2 | 1 | | | | | | | | | | |

Post-emergence

| Compound of Example No. | appln. rate lbs/A | Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|
| 38 | 1 | 1 | 1 | | 1 | 3 | 3 | 2 | 1 | 1 | 2 | 3 |
| | 2 | 1 | 1 | | 3 | 4 | 4 | 2 | 2 | 3 | 3 | 3 |
| | 4 | 3 | 2 | | 2 | 4 | 3 | 2 | 2 | 3 | 5 | 3 |
| | 8 | 2 | 1 | | 4 | | 3 | 2 | 2 | 3 | 5 | 3 |
| 39 | 1 | 1 | 2 | | 3 | 2 | 4 | 2 | 1 | 2 | 4 | 3 |
| | 2 | 3 | 2 | | 3 | 4 | 5 | 3 | 2 | 3 | 5 | 3 |
| | 4 | 4 | 2 | | 3 | 3 | 5 | 4 | 2 | 4 | 5 | 3 |
| | 8 | 3 | 2 | | 3 | 4 | 5 | 4 | 2 | 4 | 4 | 4 |
| 40 | 1 | 1 | 1 | | 3 | 2 | 5 | 2 | 1 | 1 | 2 | 1 |
| | 2 | 2 | 1 | | 4 | 3 | 5 | 2 | 1 | 1 | 3 | 1 |
| | 4 | 3 | 1 | | 4 | 3 | 5 | 3 | 1 | 2 | 3 | 3 |
| | 8 | | | | 4 | 4 | 5 | 4 | 1 | 2 | 4 | 3 |

## Table VI cont'd.

### PLANT SPECIES

#### Pre-emergence

| Compound of Example No. | appln. rate lbs/A | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia |
|---|---|---|---|---|---|---|---|---|---|---|---|---|---|---|---|---|---|---|---|---|---|
| 41 | 1 | | | | | | | | | | | | | | | | | | | | |
| | 2 | | | | | | | | | | | | | | | | | | | | |
| | 4 | | | | | | | | | | | | | | | | | | | | |
| | 8 | | | | | | | | | 1 | 1 | | 1 | 1 | 1 | 1 | 1 | 1 | | 1 | 1 |
| 42 | 8 | | | | | | | | | 3 | 1 | | 4 | 2 | 3 | 2 | 1 | 2 | | 1 | 1 |
| 43 | 1 | 1 | 1 | 1 | 1 | 1 | 1 | 1 | 1 | 1 | 1 | 1 | 4 | 1 | 3 | 1 | 1 | 1 | 1 | 1 | 1 |
| | 2 | 1 | 1 | 2 | 1 | 1 | 2 | 1 | 1 | 2 | 1 | 4 | 3 | 2 | 4 | 1 | 1 | 1 | 1 | 1 | 1 |
| | 4 | 1 | 1 | 2 | 1 | 1 | 3 | 1 | 2 | 1 | 1 | 4 | 3 | 3 | 4 | 2 | 1 | 2 | 2 | 1 | 2 |
| | 8 | | | | | | | | | 1 | 1 | | 4 | 1 | 5 | 2 | 1 | 1 | | 2 | 1 |
| 44 | 1 | 1 | 1 | 1 | 1 | 1 | 1 | 1 | 2 | 1 | 1 | 1 | 1 | 2 | 2 | 2 | 1 | 1 | 1 | 1 | 1 |
| | 2 | 1 | 1 | 1 | 1 | 1 | 1 | 1 | 1 | 1 | 1 | 4 | 2 | 2 | 2 | 3 | 1 | 1 | 3 | 2 | 2 |
| | 4 | 1 | 1 | 1 | 2 | 2 | 3 | 2 | 4 | 3 | 2 | 4 | 4 | 4 | 4 | 3 | 1 | 4 | 3 | 2 | 1 |
| | 8 | | | | | | | | | 5 | 4 | | 4 | 4 | 5 | 4 | 3 | 5 | | 3 | 4 |

#### Post-emergence

| Compound of Example No. | appln. rate lbs/A | Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|
| 41 | 1 | 2 | 1 | | 1 | 2 | 1 | 1 | 1 | 1 | 1 | 1 |
| | 2 | 2 | 1 | | 1 | 3 | 3 | 1 | 1 | 1 | 1 | 1 |
| | 4 | 2 | 1 | | 2 | 3 | 3 | 1 | 1 | 1 | 1 | 2 |
| | 8 | 3 | 1 | | 2 | 4 | 4 | 1 | 1 | 2 | 3 | 3 |
| 42 | 8 | 3 | 1 | | | 3 | | 1 | 1 | 3 | 3 | 1 |
| 43 | 1 | 1 | 1 | | | 2 | 2 | 1 | 1 | 1 | 1 | 2 |
| | 2 | 2 | 2 | | | 3 | 3 | 1 | 1 | 2 | 2 | 3 |
| | 4 | 2 | 3 | | | 3 | 3 | 3 | 1 | 2 | 3 | 3 |
| | 8 | 3 | 2 | | 3 | 5 | 4 | 4 | 2 | 4 | 4 | 3 |
| 44 | 1 | 4 | 3 | | 4 | 3 | 4 | 4 | 2 | 5 | 5 | 4 |
| | 2 | 4 | 3 | | 4 | 4 | 5 | 4 | 2 | 5 | 5 | 4 |
| | 4 | 3 | 3 | | 4 | 4 | 5 | 4 | 2 | 5 | 5 | 5 |
| | 8 | 4 | 4 | | 4 | 4 | 5 | 4 | 3 | 5 | 5 | 5 |

Table VI cont'd.

PLANT SPECIES

| Compound of Example No. | appln. rate lbs/A | Pre-emergence Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 45 | 1 | | | | | | | | | | | | | | | | | | | | | 3 | 1 | | 3 | 3 | 4 | 2 | 1 | 3 | 3 | 3 |
| | 2 | | | | | | | | | | | | | | | | | | | | | 3 | 1 | | 2 | 4 | 4 | 2 | 1 | 4 | 3 | 4 |
| | 4 | | | | | | | | | | | | | | | | | | | | | 2 | 1 | | 3 | 4 | 4 | 2 | 1 | 3 | 3 | 5 |
| | 8 | | | | | | | | | | | | | | | | | | | | | 4 | 3 | | 2 | 3 | 4 | 2 | 1 | 3 | 3 | 5 |

## Table VII

### PLANT SPECIES

| Compound of Example No. | appln. rate lbs/A | Pre-emergence | | | | | | | | | | | | | | | | | | | | Post-emergence | | | | | | | | | | |
|---|---|---|---|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Tomato | Barnyard Grass | Corn | Large Crabgrass | Mustard | Pigweed | Foxtail | Wild Oats | Velvetleaf | Morningglory | Zinnia |
| 1 | 1 | 1 | 1 | 1 | 1 | 1 | 1 | 1 | 2 | 1 | 1 | 2 | 3 | 1 | 4 | 2 | 1 | 2 | 1 | 1 | 2 | | | | | | | | | | | |
| | 0.25 | 1 | 1 | 1 | 1 | 1 | 1 | 1 | 1 | 1 | 1 | 1 | 3 | 1 | 2 | 2 | 1 | 1 | 1 | 1 | 1 | | | | | | | | | | | |
| | 0.5 | 1 | 1 | 1 | 1 | 1 | 1 | 1 | 1 | 1 | 1 | 2 | 3 | 1 | 2 | 2 | 1 | 2 | 1 | 2 | 2 | | | | | | | | | | | |
| 9 | 1 | 1 | 2 | 2 | 1 | 1 | 5 | 1 | 1 | 1 | 1 | 3 | 4 | 1 | 4 | 4 | 1 | 1 | 1 | 2 | 3 | | | | | | | | | | | |
| | 0.25 | 1 | 1 | 1 | 1 | 1 | 1 | 1 | 1 | 1 | 1 | 1 | 1 | 1 | 1 | 1 | 1 | 1 | 1 | 1 | 1 | | | | | | | | | | | |
| | 0.5 | 1 | 1 | 1 | 1 | 1 | 1 | 1 | 1 | 1 | 1 | 3 | 3 | 1 | 2 | 1 | 1 | 1 | 1 | 1 | 1 | | | | | | | | | | | |
| 11 | 1 | | | | | | | | | | | | | | | | | | | | | | | 1 | 2 | | 4 | 2 | | 2 | 2 | 3 |
| | 0.25 | | | | | | | | | | | | | | | | | | | | | | | 1 | 2 | | 4 | 2 | | 2 | 3 | 3 |
| | 0.5 | | | | | | | | | | | | | | | | | | | | | | | 1 | 2 | | 3 | 2 | | 2 | 2 | 3 |
| 12 | 1 | | | | | | | | | | | | | | | | | | | | | 2 | 1 | | 2 | 3 | 4 | 2 | 1 | 4 | 5 | 3 |
| | 0.25 | | | | | | | | | | | | | | | | | | | | | 1 | 1 | | 2 | 2 | 4 | 1 | 1 | 2 | 3 | 3 |
| | 0.5 | | | | | | | | | | | | | | | | | | | | | 2 | 1 | | 3 | 2 | 4 | 1 | 1 | 3 | 4 | 4 |
| 25 | 1 | | | | | | | | | | | | | | | | | | | | | 2 | 2 | | 4 | 4 | 4 | 3 | 2 | 4 | 5 | 4 |
| | 0.25 | | | | | | | | | | | | | | | | | | | | | 2 | 1 | | 2 | 1 | 2 | 2 | 2 | 2 | 4 | 3 |
| | 0.5 | | | | | | | | | | | | | | | | | | | | | 3 | 2 | | 3 | 3 | 3 | 2 | 3 | 2 | 4 | 3 |
| 28 | 1 | 1 | 1 | 1 | 1 | 2 | 2 | 2 | 3 | 3 | 1 | 4 | 4 | 3 | 4 | 3 | 1 | 4 | 4 | 1 | 2 | 3 | 2 | | 3 | 4 | 5 | 4 | 2 | 5 | 5 | 4 |
| | 0.5 | 1 | 1 | 1 | 1 | 1 | 1 | 2 | 1 | 1 | 1 | 2 | 1 | 2 | 3 | 2 | 2 | 2 | 1 | 1 | 2 | 3 | 2 | | 4 | 2 | 4 | 2 | 2 | 4 | 5 | 5 |

## Table VII cont'd.

### PLANT SPECIES

| Compound of Example No. | appln. rate lbs/A | Pre-emergence | | | | | | | | | | | | | | | | | | | | Post-emergence | | | | | | | | | | |
|---|---|---|---|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Tomato | Barnyard Grass | Corn | Large Crabgrass | Mustard | Pigweed | Foxtail | Wild Oats | Velvetleaf | Morningglory | Zinnia |
| 37 | 1 | | | | | | | | | | | | | | | | | | | | | 2 | 2 | | 3 | 3 | 4 | 3 | 1 | 3 | 5 | 3 |
| | 0.25 | | | | | | | | | | | | | | | | | | | | | 2 | 1 | | 2 | 2 | 4 | 2 | 1 | 2 | 5 | 3 |
| | 0.5 | | | | | | | | | | | | | | | | | | | | | 2 | 2 | | 3 | 3 | 4 | 3 | 1 | 2 | 3 | 2 |
| 38 | 1 | 1 | 1 | 1 | 1 | 1 | 1 | 1 | 1 | 1 | 1 | 3 | 2 | 1 | 1 | 1 | 1 | 3 | 1 | 1 | 1 | | | | | | | | | | | |
| | 0.25 | 1 | 1 | 1 | 1 | 1 | 1 | 1 | 1 | 1 | 1 | 2 | 1 | 1 | 1 | 1 | 1 | 1 | 1 | 1 | 1 | | | | | | | | | | | |
| | 0.5 | 1 | 1 | 1 | 1 | 1 | 1 | 1 | 1 | 1 | 1 | 4 | 4 | 1 | 2 | 2 | 1 | 2 | 1 | 1 | 1 | | | | | | | | | | | |
| 39 | 1 | | | | | | | | | | | | | | | | | | | | | 2 | 2 | | 1 | 4 | 2 | 3 | 1 | 2 | 4 | 3 |
| | 0.25 | | | | | | | | | | | | | | | | | | | | | 1 | 1 | | 1 | 3 | 3 | 1 | 1 | 1 | 2 | 2 |
| | 0.5 | | | | | | | | | | | | | | | | | | | | | 1 | 1 | | 2 | 3 | 3 | 2 | 1 | 3 | 2 | 2 |
| 40 | 1 | 1 | 2 | 1 | 1 | 1 | 1 | 1 | 1 | 1 | 1 | 3 | 1 | 2 | 1 | 1 | 1 | 2 | 1 | 1 | 1 | | | | | | | | | | | |
| | 0.25 | 1 | 1 | 1 | 1 | 1 | 1 | 1 | 1 | 1 | 1 | 4 | 1 | 1 | 1 | 2 | 1 | 1 | 1 | 1 | 1 | 1 | 2 | | 1 | 2 | 1 | 1 | 1 | 2 | 2 | 1 |
| | 0.5 | 1 | 2 | 2 | 1 | 1 | 1 | 1 | 1 | 1 | 1 | 3 | 2 | 2 | 2 | 3 | 1 | 2 | 1 | 2 | 1 | 1 | 1 | | 3 | 2 | 2 | 2 | 1 | 1 | 2 | 2 |
| 44 | 1 | | | | | | | | | | | | | | | | | | | | | 4 | 1 | | 3 | 3 | 5 | 5 | 2 | 5 | 5 | 4 |
| | 0.25 | | | | | | | | | | | | | | | | | | | | | 2 | 1 | | 2 | 2 | 3 | 2 | 1 | 3 | 5 | 3 |
| | 0.5 | | | | | | | | | | | | | | | | | | | | | 4 | 1 | | 2 | 2 | 5 | 4 | 2 | 5 | 5 | 3 |

The data generated indicates that compounds embraced by formula X are useful as terrestrial herbicides against common weed varieties which are found in desirable crops, such as corn, wheat, soybean, and the like.

Because of the potent terrestrial herbicidal activity possessed by the isothiazolylureas of formula X, they are useful in the elimination and control of the growth of unwanted terrestrial vegetation. This can be carried out by applying the herbicidal compounds by any of several art-recognized methods. The compounds can be surface applied to soil prior to planting of crops or emergence of seedlings. If desired, the surface-applied herbicide can be incorporated into the soil by conventional means. The compounds can alternatively be applied early post-emergence, for instance within about 2 weeks following seedling emergence.

The amount of herbicidal isothiazolylureas of formula X to be employed is an amount which is effective in controlling the growth of unwanted vegetation. Such herbicidal amount will depend upon a number of factors, including the method of application, formulation, soil texture, soil moisture content, the expected population of unwanted vegetation, degree of incorporation, the extent of growth control desired, and related factors. The rate of application normally will be from about 0.01 to about 10.0 pounds per acre, and preferably from about 0.25 to about 5.0 pounds per acre. (These ranges are equivalent, respectively, to from about 0.011 to about 11.2 kilograms per hectare, and from about 0.28 to about 5.6 kilograms per hectare.)

## Aquatic Herbicidal Method

A method of controlling the growth of unwanted aquatic vegetation which comprises contacting the vegetation to be controlled or the water in which the vegetation is growing with an aquatic herbicidally-effective amount of a compound of the formula

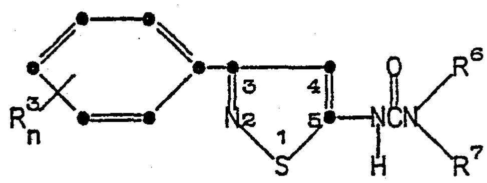

XI

wherein:

$R^3$ is hydrogen, halo, $C_1$-$C_2$ alkoxy, or $C_1$-$C_6$ alkyl;

$R^6$ and $R^7$ are $C_1$-$C_6$ alkyl, or $C_1$-$C_2$ alkoxy, provided that one of $R^6$ or $R^7$ is methyl; and

n is 1 or 2;

or an aquatically acceptable acid-addition salt thereof is also provided.

More preferred compounds of formula XI within this method are those in which:

$R^3$ is hydrogen, halo, or $C_1$-$C_4$ alkyl;

$R^6$ and $R^7$ are $C_1$-$C_2$ alkyl or $C_1$-$C_2$ alkoxy, provided that one of $R^6$ or $R^7$ is methyl; and

n is 1.

Representative preferred compounds of formula XI are:

N'-[3-(4-methylphenyl)-5-isothiazolyl]-N-methoxy-N-methylurea;

N'-[3-(4-chlorophenyl)-5-isothiazolyl]-N-methyl-N-(1-methylethyl)urea;

N'-[3-(4-fluorophenyl)-5-isothiazolyl]-N,N-dimethylurea;

N'-[3-(4-chlorophenyl)-5-isothiazolyl]-N,N-dimethylurea;

N'-(3-phenyl-5-isothiazolyl)-N,N-dimethylurea;

N'-[3-(4-chlorophenyl)-5-isothiazolyl]-N-methoxy-N-methylurea;

N'-[3-(4-methylphenyl)-5-isothiazolyl]-N-ethyl-N-methylurea;

N'-[3-(4-chlorophenyl)-5-isothiazolyl]-N-methyl-N-(1-methylpropyl)urea;

N'-[3-(4-chlorophenyl)-5-isothiazolyl]-N-methyl-N-(1-methylpropyl)urea monohydrochloride;

N'-[3-(4-fluorophenyl)-5-isothiazolyl]-N-ethyl-N-methylurea;

N'-[3-(4-fluorophenyl)-5-isothiazolyl]-N-methoxy-N-methylurea monohydrochloride;

N'-(3-phenyl-5-isothiazolyl)-N-methyl-N-(1-methylethyl)urea; and

N'-(3-phenyl-5-isothiazolyl)-N-methoxy-N-methylurea.

The compounds of formula XI for the aquatic herbicide tests were formulated in the following manner. Twenty milligrams of compound was weighed into a 12 ml disposable vial. To the vial containing the compound were added 1 ml of acetone and 9 ml of aqueous 0.1 percent polyoxyethylene sorbitan monooleate (Tween 80). This solution was then diluted with appropriate volumes of water to obtain solutions containing 10, 4, 2, 1, and 0.5 ppm (parts per million) of the test compound. The herbicidal activity was determined by visual observations based upon non-treated controls. Activity ratings were made on a scale of 1 to 5 as follows:

1 = no observable effect
2 = slight plant injury
3 = moderate plant in3ury
4 = 50-90% plant injury
5 = plants completely killed

The results of this test are reported in Tables VIII and IX which follow.

Table VIII

| Compound of Example No. | Aquatic Herbicide Activity 1-week post-treatment | | | |
|---|---|---|---|---|
| | Concentration ppm | Hydrilla | Coontail | Duckweed |
| 1 | 2 | 5 | 4 | 4 |
| | 4 | 5 | 5 | 4 |
| 2 | 2 | 2 | 4 | 1 |
| | 4 | 4 | 4 | 1 |
| 3 | 2 | 3 | 4 | 1 |
| | 4 | 2 | 2 | 2 |
| 5 | 2 | 5 | 4 | 5 |
| | 4 | 5 | 2 | 5 |
| 6 | 2 | 5 | 5 | 5 |
| | 4 | 5 | 5 | 5 |
| 11 | 2 | 5 | 2 | 3 |
| | 4 | 5 | 4 | 4 |
| 12 | 2 | 5 | 4 | 5 |
| | 4 | 5 | 5 | 5 |
| | 10 | 5 | 5 | 5 |
| 13 | 2 | 5 | 4 | 4 |
| | 4 | 5 | 5 | 5 |
| | 10 | 5 | 5 | 4 |
| 14 | 2 | 4 | 3 | 2 |
| | 4 | 5 | 3 | 2 |
| | 10 | 5 | 5 | 4 |
| 15 | 2 | 5 | 3 | 3 |
| | 4 | 5 | 4 | 4 |
| | 10 | 5 | 5 | 5 |
| 16 | 2 | 5 | 1 | 3 |
| | 4 | 3 | 2 | 3 |
| | 10 | 3 | 2 | 3 |
| 17 | 2 | 5 | 5 | 3 |
| | 4 | 5 | 4 | 4 |
| 18 | 2 | 5 | 5 | 3 |
| | 4 | 5 | 5 | 3 |

Table VIII cont'd.

| Compound of Example No. | Concentration ppm | Hydrilla | Coontail | Duckweed |
|---|---|---|---|---|
| 19 | 2 | 3 | 4 | 1 |
| | 4 | 4 | 4 | 1 |
| 20 | 2 | 3 | 4 | 2 |
| | 4 | 4 | 4 | 4 |
| 21 | 2 | 4 | 2 | 4 |
| | 4 | 4 | 1 | 4 |
| 22 | 2 | 2 | 3 | 4 |
| | 2 | 5 | - | 3 |
| | 4 | 5 | 5 | 4 |
| | 4 | 5 | - | 4 |
| | 10 | 5 | 5 | 4 |
| 23 | 2 | 5 | 4 | 4 |
| | 4 | 5 | 5 | 4 |
| 24 | 2 | 5 | 5 | 4 |
| | 2 | 1 | - | 4 |
| | 4 | 5 | 5 | 4 |
| | 4 | 5 | - | 4 |
| | 10 | 5 | 5 | 5 |
| 25 | 2 | 5 | 4 | 2 |
| | 4 | 5 | 5 | 4 |
| 26 | 2 | 5 | 5 | 4 |
| | 4 | 5 | 5 | 4 |
| 28 | 2 | 5 | 5 | 4 |
| | 4 | 5 | 5 | 4 |
| | 10 | 5 | 5 | 5 |
| 30 | 2 | 5 | 4 | 4 |
| | 4 | 5 | 4 | 5 |
| | 10 | 5 | 4 | 5 |
| 33 | 2 | 5 | 3 | 4 |
| | 4 | 5 | 4 | 4 |
| | 10 | 5 | 4 | 5 |

Table VIII cont'd.

| Compound of Example No. | Concentration ppm | Hydrilla | Coontail | Duckweed |
|---|---|---|---|---|
| 35 | 2 | 5 | 5 | 2 |
| | 4 | 5 | 5 | 3 |
| | 10 | 1 | 1 | 1 |
| 41 | 2 | 5 | 4 | 4 |
| | 4 | 5 | 5 | 4 |
| | 10 | 5 | 5 | 4 |
| 42 | 2 | 5 | 4 | 4 |
| | 2 | 5 | 4 | 4 |
| | 4 | 5 | 5 | 4 |
| | 4 | 5 | 4 | 4 |
| | 10 | 5 | 5 | 4 |
| 43 | 10 | 5 | 5 | 5 |
| 44 | 2 | 5 | 4 | 5 |
| | 4 | 5 | 5 | 5 |
| | 10 | 5 | 4 | 5 |

Table IX

Aquatic Herbicide Activity

| Compound of Example No. | Concentration ppm | 1-week post treatment: Hydrilla | Coontail | Duckweed | Naiad | Milfoil | Cabomba | Sago Pond Weed | 2-week post treatment: Hydrilla | Coontail | Duckweed | Naiad | Milfoil | Cabomba | Sago Pond Weed | 3-week post treatment: Hydrilla | Coontail | Duckweed | Naiad | Milfoil | Cabomba | Sago pond weed |
|---|---|---|---|---|---|---|---|---|---|---|---|---|---|---|---|---|---|---|---|---|---|---|
| 1 | 0.5 | 2 | 4 | 2 | 2 | 3 | 1 | 3 | 2 | 3 | 3 | 2 | 4 | 1 | 4 | 4 | 4 | 3 | 2 | 5 | 4 | 3 |
|  | 1 | 3 | 1 | 3 | 2 | 3 | 1 | 3 | 4 | 4 | 4 | 2 | 4 | 2 | 2 | 5 | 5 | 4 | 2 | 5 | 3 | 2 |
|  | 2 | 3 | 4 | 3 | 4 | 4 | 1 | 4 | 5 | 5 | 4 | 5 | 5 | 2 | 4 | 5 | 5 | 4 | 5 | 5 | 2 | 4 |
| 5 | 0.5 | 3 | 1 | 2 | 3 | 4 | 1 | 3 | 4 | 1 | 2 | 5 | 5 | 1 | 4 | 5 | 1 | 4 | 5 | 5 | 4 | 4 |
|  | 1 | 4 | 2 | 3 | 4 | 3 | 2 | 3 | 5 | 1 | 3 | 5 | 5 | 1 | 4 | 5 | 4 | 4 | 5 | 5 | 2 | 2 |
|  | 2 | 3 | 3 | 4 | 4 | 2 | 1 | 3 | 5 | 1 | 4 | 5 | 5 | 1 | 3 | 5 | 2 | 4 | 5 | 5 | 2 | 4 |
| 6 | 0.5 | 1 | 2 | 1 | 3 | 4 | 1 | 1 | 1 | 4 | 2 | 5 | 5 | 2 | 2 | 2 | 4 | 3 | 5 | 5 | 2 | 3 |
|  | 1 | 2 | 2 | 2 | 3 | 2 | 2 | 2 | 2 | 4 | 2 | 5 | 4 | 5 | 3 | 4 | 4 | 4 | 5 | 4 | 5 | 2 |
|  | 2 | 3 | 2 | 3 | 3 | 3 | 4 | 2 | 4 | 4 | 4 | 5 | 5 | 5 | 5 | 5 | 4 | 4 | 5 | 5 | 5 | 4 |
| 11 | 0.5 | 1 | 1 | 1 | 2 | 2 | 1 | 2 | 1 | 3 | 3 | 4 | 5 | 1 | 4 | 2 | 4 | 3 | 5 | 5 | 4 | 4 |
|  | 1 | 1 | 1 | 2 | 1 | 2 | 1 | 1 | 1 | 2 | 4 | 4 | 4 | 3 | 3 | 1 | 3 | 4 | 5 | 4 | 4 | 4 |
|  | 2 | 2 | 4 | 2 | 1 | 1 | 1 | 1 | 4 | 5 | 4 | 5 | 4 | 5 | 4 | 5 | 5 | 4 | 5 | 5 | 5 | 5 |
| 14 | 0.5 |  |  |  |  |  |  |  | 1 | 1 | 2 | 2 | 3 | 1 | 1 | 1 | 1 | 2 | 5 | 5 | 4 | 1 |
|  | 1 |  |  |  |  |  |  |  | 3 | 1 | 2 | 3 | 4 | 2 | 1 | 4 | 2 | 3 | 5 | 5 | 4 | 1 |
|  | 2 |  |  |  |  |  |  |  | 5 | 3 | 3 | 5 | 5 | 5 | 2 | 5 | 5 | 4 | 5 | 5 | 5 | 4 |
| 17 | 0.5 | 4 | 2 | 3 | 3 | 4 | 1 | 3 | 5 | 1 | 4 | 4 | 5 | 1 | 4 | 5 | 1 | 4 | 3 | 5 | 2 | 4 |
|  | 1 | 4 | 2 | 4 | 3 | 4 | 1 | 3 | 5 | 2 | 4 | 5 | 5 | 1 | 4 | 5 | 5 | 4 | 5 | 5 | 2 | 2 |
|  | 2 | 3 | 2 | 3 | 4 | 2 | 1 | 3 | 5 | 3 | 4 | 5 | 5 | 1 | 4 | 5 | 5 | 4 | 5 | 5 | 2 | 4 |

Table IX cont'd.

Aquatic Herbicide Activity

| Compound of Example No. | Concentration ppm | 1-week post treatment: Hydrilla | Coontail | Duckweed | Naiad | Milfoil | Cabomba | Sago Pond Weed | 2-week post treatment: Hydrilla | Coontail | Duckweed | Naiad | Milfoil | Cabomba | Sago Pond Weed | 3-week post treatment: Hydrilla | Coontail | Duckweed | Naiad | Milfoil | Cabomba | Sago pond weed |
|---|---|---|---|---|---|---|---|---|---|---|---|---|---|---|---|---|---|---|---|---|---|---|
| 18 | 0.5 | 3 | 2 | 3 | 2 | 2 | 1 | 3 | 3 | 1 | 4 | 4 | 4 | 2 | 2 | 5 | 1 | 4 | 4 | 4 | 4 | 1 |
| | 1 | 3 | 2 | 4 | 3 | 2 | 2 | 3 | 4 | 4 | 4 | 5 | 5 | 1 | 3 | 5 | 4 | 4 | 5 | 5 | 2 | 4 |
| | 2 | 4 | 2 | 3 | 3 | 5 | 1 | 3 | 5 | 2 | 3 | 5 | 5 | 1 | 4 | 5 | 3 | 4 | 5 | 5 | 1 | 4 |
| 22 | 0.5 | 1 | 1 | 1 | 3 | 3 | 2 | 1 | 2 | 1 | 1 | 4 | 4 | 5 | 1 | 3 | 1 | 1 | 5 | 4 | 5 | 3 |
| | 1 | 1 | 4 | 2 | 3 | 3 | 2 | 2 | 4 | 5 | 4 | 5 | 5 | 5 | 2 | 5 | 5 | 4 | 5 | 5 | 5 | 3 |
| | 2 | 2 | 4 | 1 | 4 | 4 | 1 | 2 | 4 | 4 | 3 | 5 | 5 | 5 | 3 | 4 | 4 | 4 | 5 | 4 | 5 | 5 |
| 23 | 0.5 | 3 | 4 | 3 | 3 | 4 | 1 | 2 | 4 | 5 | 4 | 2 | 4 | 1 | 1 | 5 | 5 | 4 | 3 | 5 | 4 | 2 |
| | 1 | 3 | 4 | 3 | 3 | 2 | 1 | 3 | 4 | 4 | 4 | 1 | 2 | 3 | 2 | 5 | 5 | 4 | 3 | 5 | 4 | 3 |
| | 2 | 4 | 1 | 4 | 4 | 2 | 1 | 3 | 5 | 1 | 4 | 5 | 5 | 3 | 4 | 5 | 3 | 4 | 5 | 5 | 5 | 4 |
| 24 | 0.5 | 2 | 1 | 1 | 1 | 2 | 2 | 2 | 4 | 1 | 2 | 5 | 3 | 4 | 1 | 5 | 1 | 3 | 5 | 3 | 5 | 2 |
| | 1 | 2 | 4 | 2 | 4 | 3 | 1 | 2 | 3 | 4 | 3 | 5 | 4 | 3 | 2 | 4 | 5 | 4 | 5 | 4 | 4 | 1 |
| | 2 | 4 | 4 | 2 | 4 | 4 | 1 | 4 | 5 | 4 | 3 | 5 | 5 | 5 | 5 | 5 | 5 | 4 | 5 | 5 | 5 | 5 |
| 28 | 0.5 | 1 | 1 | 1 | 1 | 2 | 1 | 1 | 1 | 2 | 2 | 1 | 4 | 3 | 1 | 1 | 2 | 4 | 1 | 5 | 5 | 1 |
| | 1 | 2 | 1 | 2 | 1 | 3 | 1 | 1 | 4 | 1 | 3 | 4 | 4 | 3 | 1 | 5 | 1 | 4 | 5 | 5 | 5 | 1 |
| | 2 | 1 | 1 | 2 | 1 | 3 | 1 | 1 | 4 | 2 | 3 | 5 | 5 | 5 | 1 | 5 | 2 | 4 | 5 | 5 | 5 | 1 |
| 35 | 0.5 | 1 | 4 | 2 | 1 | 1 | 1 | - | 1 | 4 | 2 | 1 | 3 | 1 | - | 1 | 4 | 4 | 2 | 3 | 5 | - |
| | 1 | 1 | 4 | 2 | 2 | 2 | 1 | - | 1 | 4 | 3 | 1 | 1 | 1 | - | 3 | 4 | 4 | 5 | 5 | 5 | - |
| | 2 | 2 | 4 | 3 | 2 | 1 | 1 | - | 2 | 4 | 4 | 5 | 4 | 2 | - | 3 | 4 | 4 | 5 | 5 | 5 | - |

Table IX . cont'd.

Aquatic Herbicide Activity

| Compound of Example No. | Concentration ppm | 1-week post treatment: Hydrilla | Coontail | Duckweed | Naiad | Milfoil | Cabomba | Sago Pond Weed | 2-week post treatment: Hydrilla | Coontail | Duckweed | Naiad | Milfoil | Cabomba | Sago Pond Weed | 3-week post treatment: Hydrilla | Coontail | Duckweed | Naiad | Milfoil | Cabomba | Sago pond weed |
|---|---|---|---|---|---|---|---|---|---|---|---|---|---|---|---|---|---|---|---|---|---|---|
| 44 | 0.5 | 1 | 1 | 1 | 1 | 1 | 1 | 1 | 3 | 2 | 2 | 4 | 5 | 5 | 2 | 4 | 3 | 4 | 5 | 5 | 5 | 3 |
| | 1 | 1 | 1 | 1 | 1 | 1 | 2 | 1 | 3 | 2 | 3 | 5 | 5 | 5 | 3 | 3 | 4 | 5 | 5 | 5 | 5 | 4 |
| | 2 | 1 | 1 | 1 | 1 | 1 | 2 | 2 | 5 | 3 | 4 | 5 | 5 | 5 | 4 | 5 | 5 | 5 | 5 | 5 | 5 | 5 |
| 45 | 0.5 | 1 | 2 | 1 | 1 | 2 | 1 | 1 | 3 | 2 | 2 | 5 | 4 | 3 | 3 | 5 | 3 | 2 | 5 | 4 | 3 | 1 |
| | 1 | 1 | 1 | 1 | 3 | 2 | 1 | 1 | 5 | 3 | 2 | 5 | 5 | 3 | 3 | 5 | 4 | 3 | 5 | 5 | 5 | 3 |
| | 2 | 2 | 2 | 1 | 5 | 4 | 1 | 1 | 5 | 3 | 3 | 5 | 5 | 4 | 3 | 5 | 4 | 3 | 5 | 5 | 5 | 5 |

Formulations

Also provided is a formulation comprising a compound of the formula IXA, X, or XI together with an agriculturally- or aquatically-acceptable carrier.

The aquatic herbicidal method and the algicidal method are practiced by adding the active isothiazolylureas of formula XI or IXA to the water containing the submerged, emergent, ditchbank, or floating aquatic plants or algae or otherwise contacting aquatic plants with the active compounds, for example, by applying the compounds to the sub-aqueous soil in which the aquatic plants are rooted. The formulations suitably contain from about 5 to about 90 percent by weight of the active ingredient. The compounds may also be mixed with surface-active dispersing agents to form concentrates to facilitate dispersion in water and to improve the wetting properties when used as sprays.

If desired, the compounds of formula XI or IXA may be mixed with a powdered solid carrier such as bentonite, Fuller's earth, diatomaceous earth, or various mineral silicates, and clays, together with surface-active wetting and dispersing agents, so that a wettable powder may be obtained which may be applied directly, or which may be mixed with water to make an aqueous dispersion for application in that form. These wettable powder formulations suitably contain from about 25 to about 85 percent by weight of the active ingredient. The compounds may be dissolved in an oil, such as a hydrocarbon or chlorinated hydrocarbon oil, and the oil solution of the compound dispersed in water with the

aid of a surface-active emulsifying agent to give a sprayable aqueous emulsion. These surface-active emulsifying agents may be anionic, nonionic, or cationic surface-active agents. Such surface-active agents are well-known, and reference is made to Hoffman et al., U.S. Patent No. 2,614,916, columns 2-4, for detailed examples of the same.

Further, the compounds can also be applied in an invert emulsion formulation. An invert emulsion formulation is prepared by first making a solution of the compound in heavy oils, such as diesel fuel, inverting oil, and the like, and combining the thus-obtained solution with water under high shear stirring. Densifying agents, such as powdered limestone or iron oxide, may be needed to increase the density of the invert. The thick emulsion is placed in the water and sinks to the bottom of the lake, river, pond, or the like, and the aquatic herbicide or algicide is gradually released to control the growth of the aquatic plants or algae. The following is an example of an invert emulsion formulation.

Invert Emulsion

| Ingredient | Amount |
|---|---|
| Compound of Example 37 | 12.5 g |
| Diesel fuel | 333 ml |
| Inverting oil* | 333 ml |

*Visko-Rhap Inverting Oil (Rhodia, Inc.)

Two-hundred fifty milliliters of this solution is combined with 3750 ml of water under high shear stirring to give a thick invert emulsion.

The compounds useful as aquatic herbicides or algicides can also be applied as pellets, which are prepared from a mixture of about 5% of the active ingredient, about 87% balling clay, and about 10% water, all percentages being by weight. The mixture is then extruded through a pellet mill using a suitably sized die, e.g., about 1/8 in. (0.32 cm) diameter. The extruded pellets are about 1/8 in. (0.32 cm) by 1 1/2 in. (3.81 cm), and are then dried to about 8% moisture content.

The method of controlling aquatic weeds or algae is practiced by adding to the water containing the submerged, emergent, ditchbank, or floating plants or algae, a herbicidal or algicidal amount of one of the compounds of formula XI or IXA, such that a concentration of from about 0.01 to about 10 ppm of the active compound is attained.

The optimum concentration of active compound of formula XI or IXA for any specific aquatic control problem varies with the temperature, the species to be controlled, and the shape of the body of water to be treated. At higher water temperatures, less compound is generally required for a given degree of control than is needed at lower temperatures. When used to control algae or aquatic weeds, the compounds will usually be employed at concentrations of about 0.1 to about 10 ppm. In terms of pounds of compound per acre of water one foot deep, 0.1 to 10 ppm is equal to about 0.3 to about 30 pounds per acre of water one foot deep (33.6 kg/ha of water 0.3 m deep).

In considering the treatment of moving streams for the purpose of controlling vegetation fixed therein, special account must be taken of the fact that the compounds will pass over the area to be treated and that the concentration during the contact period is dependent upon the water flow rate, the rate of chemical addition, and the time period of addition.

The isothiazolylurea herbicides of formula X to be employed according to the terrestrial method can be formulated for convenient application as aqueous sprays, solid pellets, or granules, and the like. Herbicidal formulations containing an isothiazolylurea of formula X will contain from about 1.0 to about 90 percent by weight of active isothiazolylurea, and can be prepared by combining the isothiazolylurea with any number of common herbicidal carriers, diluents, adjuvants, and the like. The formulations can contain liquid carriers and adjuvants such as organic solvents, including benzene, xylene, toluene, a halotoluene such as ortho-chlorotoluene, n-hexane, n-pentane, kerosene, and similar organic solvents. The formulations can additionally contain art-known emulsifiers, stabilizers, dispersants, suspending agents, spreaders, penetrants, wetting agents, and the like.

Typical carriers utilized in the dry formulations include clay, diatomaceous earth, silica, pyrophyllite, and the like. Herbicidal powders are prepared by grinding and blending such solid carriers with a compound of formula X. Granular formulations provided herein are prepared by impregnating an isothiazolylurea

onto or into a granulated carrier such as a vermiculite. Such granules typically have a particle size of about 0.2 to about 2.0 mm.

Preferred formulations are in the form of aqueous suspension concentrates (flowables). Such formulations employ any number of commercially available wetting agents, and dispersing agents, such as ethoxylated nonyl phenols, ethoxylated octyl phenols, polyoxyethylene stearyl ethers, lignin sulfonates, blends of such agents, and the like. Such formulations can optionally contain adjuvants: such as thickening agents, for instance heteropolysaccharide gums and the like; such as xanthan gum; as well as antibacterial agents, such as aqueous formaldehyde.

The following detailed examples of formulations are given as illustrations.

Granule

| Ingredient | Percent by weight |
|---|---|
| Compound of Example 1 | 5.0 |
| Clay granule | 95.0 |

The compound is substantially dissolved in acetone or similar solvent, and the organic solution is sprayed onto the clay, which is in the form of chips. The mixture is then thoroughly blended and dried.

Wettable Powder

| Ingredient | Percent by weight |
| --- | --- |
| Compound of Example 29 | 75.0 |
| Fuller's earth | 19.0 |
| Sulfonated lignin | 3.5 |
| Sodium lauryl sulfate | 2.5 |

Wettable Powder (50 W)

| Ingredient | Percent by Weight |
| --- | --- |
| Compound of Example 24 | 51.5 |
| Wetting agent | 5.0 |
| Dispersing agent | 5.0 |
| Anticaking agent | 5.0 |
| Barden clay | 33.5 |

Wettable Powder (50 W)

| Ingredient | Percent by Weight |
| --- | --- |
| Compound of Example 32 | 51.5 |
| Wetting and dispersing agent | 8.0 |
| Anticaking agent | 3.0 |
| Barden clay | 37.5 |

Examples of the various classes of ingredients are: wetting agent--naphthalene sulfonate (Sellogen HR by Westvaco Chemical); dispersing agent--sulfonated lignin (Polyfon O by Westvaco Chemical); anticaking agent--$SiO_2 \cdot H_2O$--(Zeolex 7 by Hubler Chemical); wetting and dispersing agent--(Reax 45L by Westvaco Chemical); carrier--(Barden clay by Hubler Chemical). Other

suitable wetting agents, dispersing agents, anticaking agents or carriers discussed above can also be employed.

The above ingredients for each formulation are blended to uniformity and are ground in a hammer mill or air mill. The product is then reblended to a homogenous free flowing powder. The powder is dispersed in water and sprayed onto the weed-infested area.

## CLAIMS

1. A urea compound of the formula

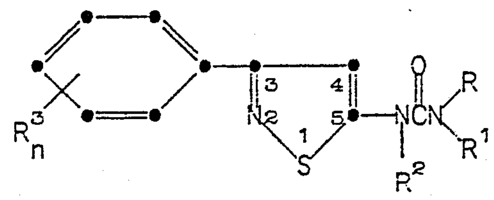

IX

wherein

R and $R^1$ are $C_1$-$C_6$ alkyl, $C_3$-$C_6$ alkenyl, or $C_1$-$C_2$ alkoxy, provided that one of R or $R^1$ is methyl;

$R^2$ is hydrogen or $C_1$-$C_3$ alkyl;

$R^3$ is hydrogen, halo, $C_1$-$C_2$ alkoxy, or $C_1$-$C_6$ alkyl; and

n is 1 or 2;

or an agriculturally- or aquatically-acceptable acid-addition salt thereof.

2. The compound of Claim 1 wherein

R and $R^1$ are $C_1$-$C_4$ alkyl or $C_1$-$C_2$ alkoxy;

$R^2$ is hydrogen;

$R^3$ is hydrogen, halo, or methyl; and

n is 1.

3. Any one of the following compounds, or an agriculturally- or aquatically-acceptable acid-addition salt thereof:

N'-[3-(4-methylphenyl)-5-isothiazolyl]-N,N-dimethylurea

N'-(3-phenyl-5-isothiazolyl)-N-methoxy-N-methylurea

N'-[3-(4-fluorophenyl)-5-isothiazolyl]-N-methoxy-N-methylurea

N'-[3-(4-fluorophenyl)-5-isothiazolyl]-N-methoxy-N-methylurea monohydrochloride

N'-[3-(4-fluorophenyl)-5-isothiazolyl]-N,N-dimethylurea.

4. A process for preparing the compounds of formula IX, as defined in claim 1, which comprises reacting a 5-aminoisothiazole of the formula

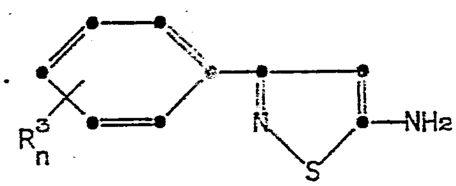

V

wherein $R^3$ and n are defined as in Claim 1, with a ureido forming reagent, to provide the compounds of formula IX wherein $R^2$ is hydrogen;

followed by alkylation with

$R^{2'}Y$

wherein $R^{2'}$ is $C_1$-$C_3$ alkyl and Y is bromo, chloro, fluoro or iodo, to provide the compounds of formula IX wherein $R^2$ is $C_1$-$C_3$ alkyl; and

optionally followed by salification to form the corresponding acid-addition salt.

5. The use of a urea derivative of the formula

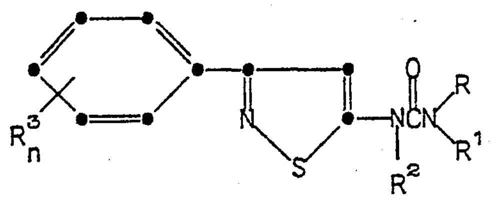

IXA

wherein R, $R^1$, $R^2$, $R^3$ and n are defined as in claim 1, or an aquatically-acceptable acid-addition salt thereof;

provided that when one of R and $R^1$ is methyl and the other is 1-methylethyl, and $R^2$ is hydrogen, $R^3$ cannot be 4-fluoro; an aquatic algicide

6. The use of a urea derivative of the formula

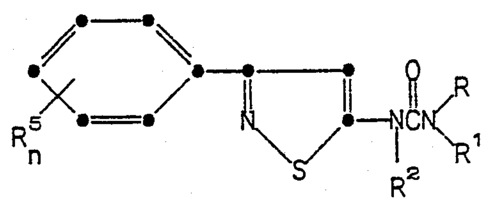

X

wherein R, $R^1$, $R^2$ and n are defined as in claim 1,

$R^5$ is hydrogen, halo, methoxy, or $C_1$-$C_3$ alkyl; or an agriculturally-acceptable acid-addition salt thereof;

provided that when R and $R^1$ are methyl, and $R^2$ is hydrogen, $R^5$ cannot be 3-methyl; as a terrestrial herbicide.

7. The use of urea derivative of the formula

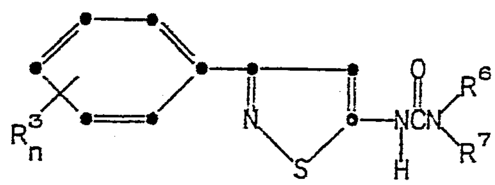

XI

wherein $R^3$ and n are defined as in claim 1, $R^6$ and $R^7$ are $C_1$-$C_6$ alkyl, or $C_1$-$C_2$ alkoxy, provided that one of $R^6$ or $R^7$ is methyl; or an aquatically-acceptable acid-addition salt thereof; as an equatic herbicide

8. An aquatic algicidal formulation comprising as an active ingredient a compound of formula IXA, as defined in claim 5, or an aquatically-acceptable acid-addition salt thereof, associated with one or more aquatically-acceptable carriers or diluents therefor.

9. A terrestrial herbicidal formulation comprising as an active ingredient a compound of formula X, as defined in claim 6, or an agriculturally-acceptable acid-addition salt thereof, associated with one or more agriculturally-acceptable carriers or diluents therefor.

10. An aquatic herbicidal formulation comprising as an active ingredient a compound of formula XI, as defined in claim 7, or an aquatically-acceptable acid-addition salt thereof, associated with one or more aquatically-acceptable carriers or diluents therefor.